# EUROPEAN PATENT SPECIFICATION

(11) **EP 0 396 964 B1**
(45) Date of publication and mention of the grant of the patent: **06.09.1995**
(21) Application number: 90107892.3
(22) Date of filing: 25.04.1990
(51) Int. Cl.: A61K 39/10, C12N 15/31

(54) **Pertussis toxin mutants, bordetella strains capable of producing such mutants and their use in the development of antipertussis vaccines**
Pertussistoxin-Mutanten, dieselbe produzierende Bordetella-Stämme und ihre Verwendung als Vakzin gegen Pertussis
Mutants de la toxine pertussis, souches de bordetella les produisant et leur utilisation comme vaccins contre pertussis

(30) Priority: 28.04.1989 IT 2034189; 07.02.1990 IT 1928690
(43) Date of publication of application: 14.11.1990
(73) Proprietor: SCLAVO S.p.A., I-53100 Siena (IT)
(72) Inventor: Pizza, Mariagrazia, I-53100 Siena (IT); Covacci, Antonello, I-53100 Siena (IT); Rappuoli, Rino, I-53035 Quercegrossa/Monteriggioni Siena (IT); Nencioni, Luciano, I-53036 Poggibonsi (Siena) (IT)
(74) Representative: Hallybone, Huw George

(56) References cited:
- EP-A- 47 802
- EP-A- 0 306 318
- EP-A- 0 320 866
- EP-A- 0 322 115
- EP-A- 0 322 533
- EP-A- 0 352 250
- SCIENCE, vol. 242, no. 4875, 1988, pages 72-74; W.N. BURNETTE et al.: "Pertussis toxin S1 mutant with reduced enzyme acitvity and a conserved protective epitope"
- SYMP. ON MOLECULAR AND CELLULAR BIOLOGY, 17th - 22nd January 1989, J. CHEM. BIOL. SUPPL. 0 (13 part A), 1989, page 67, abstract no. A 210; S. COCKLE et al.: "Inactivation of pertussis toxin by site-directed mutagenesis"
- INFECTION AND IMMUNITY, vol. 56, no. 8, August 1988, pages 1934-1941, American Society for Microbiology; J.T. BARBIERI et al.: "ADP-Ribosyltransferase mutations in the catalytic S-1 subunit of pertussis toxin"
- PROC. NATL. ACAD. SCI. USA, vol. 85, October 1988, pages 7521-7525; M. PIZZA et al.: "Subunit S1 of pertussis toxin: Mapping of the regions essential for ADP-ribosyltransferase activity"
- SCIENCE, vol. 246, 27th October 1989, pages 497-500; M. PIZZA et al.: "Mutants of pertussis toxin suitable for vaccine development"

## Description

The present invention refers to new, immunologically active pertussis toxin strains having reduced or no toxicity, capable of producing and secreting said pertussis toxin mutants, means and methods for their preparation, and their use for developing effective antipertussis vaccines.

The present invention also refers to immunogenic formulations suitable as antipertussis vaccines containing as an active component at least one immunogenically active pertussis toxin mutant having reduced or no toxicity, which may be treated with formaldehyde, or a Bordetella strain capable of producing and secreting said mutant, or a Δ tox Bordetella strain incapable of producing the pertussis toxin.

Pertussis, an infectious disease of bacterial origin characterized by a convulsive cough and serious respiratory symptomatology, affects individuals of all ages and, in the first years of life, is lethal in 0.5% of cases.

Bordetella pertussis (B. pertussis), which is the etiological agent of pertussis, produces during the virulent stage (stage I) a series of toxic components among which the pertussis toxin (PT) represents not only the principal pathogenic agent of the disease but also the major immunogen.

PT, which has the structure of a hexamer consisting of five different subunits (S1, S2, S3, S4 and S5) is capable of inducing in experimental animals antibody levels sufficient to impart protection against pertussis.

The incidence of infection may be controlled by immunization of an individual with a suitable vaccine.

At present, a cellular vaccine is employed, that is a vaccine consisting of whole cells of virulent *B. pertussis* treated with merthiolate and killed at 56°C.

Said vaccine, although imparting a protective immunity, may produce, however, undesirable side effects ranging from simple pompholyx, erythema and fever to convulsions and cerebral damage. For these reasons, the use of said vaccine has been drastically reduced in the last few years, resulting in new outbreaks of the disease.

Acellular vaccines were therefore proposed in the art consisting of one or more antigenic, toxic proteins produced and secreted by virulent *B. pertussis* detoxified with a variety of chemicals reagents such as formaldehyde (Sato *et al*. (1983), Infect. Immun., 41, 313-320), glutaraldehyde (Quentin-Millet *et al*. (1988) J. Bio. Stand., 16, 99-108), tetranitromethane (Siber *et al*. 1988; Windberry *et al*., 1988, International Workshop of *Bordetella pertussis*, Hamilton, MO), trinitrobenzensulfonic acid (Fisch *et al*., 1984, Infect. Immun, 44, 1-16), and hydrogen peroxide (Sekura *et al*. (1983), Infect. Immun., 113:806-813).

These detoxification methods present, however, the following drawbacks:
1) reversion of protein toxicity. In fact, acellular vaccines consisting only of formaldehyde detoxified PT or of PT and filamentous hemagglutinin (FHA) both treated with formaldehyde (Sato Y. *et al*. (Lancet 1, 122-126, 1984), although being capable of protecting 80% of children from disease and 50 to 60% from infection (Ad hoc Group for the Study of Pertussis Vaccines, 1988, Lancet 1, 959-960), show a reversion of toxicity (Sortsaeter J. *et al*., Pediatr. Infect. Dis. J., 7, 637-645, 1988).
2) reduced immunogenicity of the antigenic proteins caused by the drastic conditions required in the detoxification stage;
3) absence of reproducibility of the detoxified products;
4) necessity of tests to evaluate the reversion for each preparation, tests which require a long time; and finally
5) risks in handling large amounts of toxic material for people employed in the preparation of such antigenic proteins.

The toxicity of the pertussis toxin is mediated by the ADP-ribosyl-transferase activity of its S1 subunit. In order to obtain molecules with an altered toxicity with respect to the wild type pertussis toxin (PT), suitable for the preparation of pertussis vaccines free of the above mentioned drawbacks, a series of deletion mutants of the N-terminal and/or C-terminal portion of S1 were constructed and expressed in *Escherichia coli (E. coli)*, as well as a series of peptides, analogous to S1, containing in their sequence one or more amino acid substitutions, as disclosed in EP-A-0 322 533.

In practice, the DNA fragment encoding subunit S1 of PT was modified, by site-directed mutagenesis, to encode a sub-unit containing, at specific sites, an amino acid residue different from the one present in PT. The peptides obtained through culture of said engineered *E. coli* strains showed an altered toxicity with respect to the corresponding pertussis toxin peptide. Said peptides, however, were expressed as proteins fused to aminoterminal sequence of 98 amino acids of bacteriophage MS2 polymerase.

Furthermore, when tested *in vivo* (mice), said peptides were incapable of inducing the formation of protective antipertussis antibodies, probably for the reason that they could not show the same conformational structure that they assume in the native molecule. Therefore, processes employing host microorganisms such as *E. coli* engineered by means of recombinant DNA techniques to obtain heterologous proteins (that is proteins that are not naturally produced by said host strains) do not appear suitable for the preparation of products having the desired immunogenic properties.

Pizza *et al*. (PNAS USA (1988), 85, 7521-7525) describes the mutation of the pertussis S1 unit by C- and N-terminal deletion and by site-directed mutagenesis at single and multiple sites to produce non-toxic mutants.

Barberi *et al*. (Inf. Imm. (1988), 56(8), 1934-1941) describes single amino acid substitutions of Asp11, Arg13, Trp26 and Glu139 of the S1 sub-unit, each of which is said to reduce the enzymatic activity of the sub-unit.

EP-A-0 306 318 describes mutations in the region of the S1 sub-unit bounded by Va17 and Pro14.

EP-A-0 322 115 (which was unpublished at the priority date hereof) describes various single and multiple amino acid substitutions in the S1 unit said to reduce toxicity.

EP-A-0 047 802 describes the use of formaldehyde as a detoxifying treatment for pertussis toxin.

An objective of the present invention is to obtain immunogens suitable for the preparation of antipertussis vaccines devoid of the setbacks of the prior art.

This is obtained according to the present invention by providing new Bordetella strains capable of expressing and secreting pertussis toxin mutants with reduced or no toxicity.

An object of the present invention is therefore to obtain immunogenically active mutants having reduced or no toxicity, characterised in that the amino acid sequence of the S1 subunit comprises, at specific sites, one or more deleted residues or residues substituted by a different amino acid residue.

A further object of the present invention is immunogenically active pertussis toxin mutants, free of toxicity or having reduced toxicity, characterised by thermal stability and by reduced or absent mitogenetic and hemagglutination properties, obtained by treatment with a wt/vol percentage of formaldehyde of between 0.035% and 0.420%.

A still further object of the present invention is Bordetella strains capable of producing and secreting immunogenically active pertussis toxin mutants having reduced or no toxicity.

Another objective of the present invention is a method for preparing such Bordetella strains.

Still another object of the present invention is a process for the preparation of immunogenically active pertussis toxin mutants with reduced or no toxicity, which comprises cultivating in suitable conditions such mutated Bordetella strains.

A further object of the present invention is the use of Bordetella strains capable of producing and secreting immunogenically active mutants of the pertussis toxin showing reduced or no toxicity, and/or Δ tox Bordetella strains incapable of producing pertussis toxin, for the preparation of effective antipertussis cellular vaccines.

A further object of the present invention is the use of immunogenically active pertussis toxin mutants with reduced or no toxicity optionally treated with formaldehyde for the preparation of effective antipertussis acellular vaccines.

The present invention has furthermore as an object immunogenic formulations suitable as antipertussis vaccines capable of inducing in humans an effective protective response against infections deriving from virulent *B. pertussis*, containing an immunogenically effective amount of a Bordetella strain as above defined.

A still further object of the present invention is immunogenic formulations suitable as antipertussis vaccines capable of producing in humans an effective protective response against infections deriving from virulent Bordetella pertussis, containing an immunogenically effective amount of an immunogenically active pertussis toxin mutant having reduced or no toxicity, said mutant being optionally treated with formaldehyde.

Further objective of the present invention will be evidenced by reading the description and examples that follow.

According to the present invention, there is provided an immunologically active pertussis toxin mutant having reduced or no toxicity characterised in that amino acid residue Glu129 and an animo acid residue or combination of residues selected from Arg9, Asp11, Arg13, Trp26, Phe50, Gly86, Ile88/Tyr89, Asp11/Trp26, Asp11/Phe50, Arg13/Trp26, Arg13/Phe50 and Tyr8/Arg9 are deleted or substituted by a different amino acid residue selected from the group of natural amino acids, excluding mutants having an Arg9 or Arg13 deletion or an Arg9 or Arg13 to Glu substitution in combination with a Glu129 or Glu129/Tyr130 substitution.

Preferred pertussis toxin mutants according to the present invention are the ones characterised by the fact that amino acid residue Glu129 and at least one of the amino acid residues Art9, Asp11, Asp13 and Trp26 are deleted or substituted by a different amino acid residue selected from the group of natural amino acids.

According to an embodiment of the present invention the pertussis toxin mutants contain the amino acid substitutions reported in Table II, column 1 where:
- in the first line, the name of the mutated protein is reported;
- in the second line the type of mutation performed is reported and
- in the third line the nucleotide sequence utilized for the mutation is reported.

Particularly preferred among these are the pertussis toxin mutants designated as follows:
(PT-26I/129G), L13/I26/28G (PT-13L/26I/129G), PT-88E/89S and E88/S89/28G (PT-88E/89S/129G).

PT mutants having the above listed characteristics are obtained, according to the present invention, by cultivation of Bordetella strains containing a chromosomal gene encoding for PT isolated from B. pertussis mutated by site-directed mutagenesis or by deletion of nucleotides in one or more specific sites of the nucleotide sequence encoding the S1 subunit.

According to the present invention said Bordetella strains are obtained by a process comprising:
a) selection of wild type Bordetella strains resistant to at least one antibiotic;
b) substitution, through homologous recombination in the strains obtained in a) of the chromosomal gene encoding pertussis toxin with a gene encoding a different protein;
c) selection of Bordetella strains devoid of the PT (Δ tox) gene obtained in b);
d) mutation of the pertussis toxin gene isolated from *B. pertussis*;
e) introduction of the mutated gene into a suitably modified plasmid non replicable in Bordetella;
f) introduction by conjugation of said plasmid in the Bordetella (Δ tox) strains selected in c) and finally
g) isolation of Bordetella strains in which homologous recombination has taken place with the mutated pertussis toxin gene.

Bordetella wild type strains according to the present invention are selected from the species *B. pertussis*, *B. parapertussis* and *B. bronchiseptica*. The last two, although possessing the pertussis toxin operon, do not produce it normally because of the absence of a functional promoter.

In stage a) of the process of the present invention, Bordetella strains are made resistant to one or more antibiotics in order to facilitate selection of the mutated strains.

According to an embodiment of the present invention, said Bordetella strains are made resistant to nalidixic acid (nal) and to streptomycin (str).

In stage b) of the process according to the present invention the substitution is performed by homologous recombination of the chromosomal gene encoding the PT contained in the strains obtained as in a), with a gene encoding a protein different from PT, for instance kanamycin resistance (kan). The recombination may be performed employing generally known techniques, employing a plasmid non replicable in Bordetella. Preferably plasmid pRTP1 is employed, the construction of which was described by Stibitz S. *et al*. (GENE, 50, 133-140, 1986).

Said plasmid may be introduced into Bordetella by conjugation with two components using a *E. coli* strain, or with three components using a *E. coli* strain containing a so-called helper plasmid. According to the present invention, the pRTP1 plasmid is digested with the EcoRI restriction enzyme and then ligated with a DNA EcoRI fragment containing the gene that encodes for a protein different from PT and comprised among the nucleotide sequences corresponding to regions 1-420 and 3625-4696 of the *B. pertussis* PT gene contained in the PT101 ATCC 67854 plasmid. *E. coli* cells are then transformed with the resulting plasmid, and the transformants are selected employing conventional techniques.

The thus selected positive clones are then conjugated with the Bordetella strains obtained in a), previously cultivated on Bordet-Gengou (BG) medium at 37°C for about 48 hours. The conjugation is performed, according to conventional techniques, on BG medium with added 10 mM MgCl₂ at 37°C for 3-6 hours.

In stage c) of the process according to the present invention the Bordetella strains, in which homologous recombination at chromosomal level has taken place, are selected on BG medium made selective by the addition of suitable antibiotics. When nal and str resistant Bordetella strains are employed and the gene different from PT is the kanamycin gene, the antibiotics added to the medium are nal, str and kan.

The strains that grow on this medium (resistant to the three antibiotics) are the ones in which the complete substitution of the PT gene by kanamycin gene has taken place and which have lost the pRTP1 plasmid which imparts sensitivity to streptomycin.

For the purpose of confirming such substitution said strains, indicated in what follows as Δ tox, were characterised by means of Southern blotting (E. Southern, J. Mol. Biol. (1975) 98, 503-517), ELISA assay (Wong, K.H. and Skelton S.K.J. Clinical Microbiol. Vol. 26, 1316-1320, 1988) and toxicity test on CHO cells (Hewlett, E.L. *et al*. (1983) Infect. Immun. 40, 1198-1230).

The results have shown:
a) the presence in the Δ tox strain chromosomal DNA of a nucleotide fragment with a molecular weight lower than that of the PT gene, which hybridizes both with said gene and with its substituent (kanamycin gene);
b) none of the Δ tox strains was capable of producing and secreting pertussis toxin in an amount detectable by the ELISA assay;
c) toxicity on CHO cells, determined employing the supernatant of Bordetella Δ tox diluted 1/10 is insufficient to modify their growth. A slight, non specific toxicity was observed employing the pure supernatant.

For the purpose of ascertaining the capacity of said Δ tox strains to impart protection against virulent *B. pertussis* an "intracerebral challenge" assay was performed as described in "21/PAR7620.4 CODE OF FEDERAL REGULATIONS, Potency test of pertussis vaccine". The obtained results, reported in Example 1, showed that said strains, although no longer possessing the PT encoding gene, are still capable of inducing excellent protection against intracerebral infections due to virulent *B. pertussis*.

In stage d) of the process of the present invention, the construction of the mutagenized PT gene was performed by deletion or substitution, through site-directed mutagenesis, of one or more nucleotides in determined positions of the nucleotide sequence which encode for the S1 subunit of the gene encoding the *B. pertussis* PT contained in the PT101 ATCC 67854 plasmid.

According to one embodiment of the present invention, PT genes are constructed containing the mutations reported in Table II, utilizing the nucleotide sequences listed in line 3 of the first column.

In stage e) of the process of the present invention, the mutated genes obtained in stage d) are cloned in a plasmid non replicable in Bordetella.

To that end, plasmid pRTP1 was utilized, modifying it by insertion into its BamHI restriction site, the gene encoding for resistance to gentamycin or the one encoding for resistance to tetracycline (both commercially available). Cloning such genes was performed according to one of the known techniques generally employed in genetic engineering. The new vectors, indicated respectively as pRTPG1 and pRTPT1 were thus employed to insert the mutated PT gene into the Bordetella Δ tox strain's chromosome.

In particular, the mutated genes were cloned into plasmids pRTPG1 and pRTPT1 and the resulting recombinant plasmids were introduced, by transformation, into *E. coli* cells. The transformants are conjugated with Δ tox Bordetella strains as described.

*E. coli* cells suitable for the purposes of the present invention are *E. coli* SM10 described by Simon R.*et al* Biotech. 1, 784-791, 1983.

Finally, in stage g) of the process of the present invention the selection of Bordetella strains containing in their chromosome the mutated PT gene is performed.

In particular, first the selection of Bordetella strains which contain the recombinant plasmid integrated in the chromosome is performed, by cultivating on BG medium with added nal and gentamicin or nal and tetracycline, and then the selection of strains which have lost said plasmid, by cultivation on Bg medium containing str. Finally, the colonies capable of growing on this medium were isolated and cultivated on BG medium containing nal, str and kan or nal and str. Operating in this way on this last medium, Bordetella colonies were selected which have lost the kanamycin resistant phenotype because of the substituted of kan gene with the mutated PT gene.

In order to ascertain the capacity of said Bordetella strains to express and secrete the PT mutants encoded by the mutated chromosomal gene, some of these strains were cultivated in a suitable medium, such as for instance the medium having the composition reported in Example 1. The production data showed:
1) the *B. pertussis* strains produced the PT mutants in amounts comparable to the ones obtained by cultivating the same wild type strains;
2) the *B. bronchiseptica* and *B. parapertussis* strains which do not normally produce the PT toxin, are surprisingly capable of producing and secreting it into the culture medium, and
3) the *B. parapertussis* strain produces PT in higher amounts than *B. pertussis*.

Said results showed that the substitution of the inactive promoter, present in said wild type Bordetella, by an efficient promoter such as, for example, the one of the *B. pertussis* PT, allowed the expression in said strains of PT or of mutants of the same.

According to the present invention, the PT mutants obtained as described are purified from cell-free medium utilizing purification techniques selected from the ones known to the expert in the field, such e.g. the one described by Sekura R.D. *et al*, J. Biol. Chem. 258, 14647-14651 (1983).

According to the present invention, the physicochemical, biological and immunological properties of certain PT mutants were determinated *in vitro* and *in vivo*.

As far as the physicochemical properties are concerned, analysis by electrophoresis in SDS on polyacrylamide gel (SDS-PAGE) showed the absence of contaminant proteins and a pattern identical to that of PT, while the amino acid analysis showed an amino acid composition in agreement with the values predicted on the basis of the known amino acid sequence.

Furthermore, the absence of dimethyl (2,6-0-) betacyclodextrin was confirmed employing the method described by Beley J.G. (1985), "Laboratory techniques in biochemistry and molecular biology". Burdon R.H. and Van Knippennberg P.H. (ed.) Elsevier, vol. 16. The absence of fetuin, protein 69 KD an filamentous hemagglutinin, which are the possible contaminants of acellular antipertussis vaccines, was confirmed by means of Western Blotting analysis (Towbin H.T. *et al* (1976), P.N.A.S., U.S.A. 73, 361-365) utilizing antibodies specific for such proteins. Finally, the absence of thermonecrotic toxin was confirmed by means of the assay performed on guinea pigs as described by Kime K.T. *et al*., (1986), (Infect. Immun. 52, 370-377), while the absence of cyclodextrin which is the prevalent component of the culture medium, was proved by thin layer chromatography.

The absence or reduction of the PT mutants' toxicity according to the present invention is determined in various experimental systems *in vitro* and *in vivo*.

The results obtained in the CHO (Chinese Hamster Ovary) cells toxicity assay showed a reduction down to disappearance of the toxicity with respect to PT native toxicity, of from 10 to 1.000,000 times. In particular, the best results were obtained using the PT-129G, PT-9K/129G, PT-13L/129G, PT-26I/129G, PT-88E/89S and PT-88E/89S/129G mutants.

Furthermore, in none of the other assays was any toxicity of the product observed at the maximum employed doses.

Such results confirm that all the toxic PT activities are due to the ADP-ribosyltransferase activity of its S1 subunit.

The only PT mutants' activities which were not altered by the genetic manipulations of the S1 subunit were the mitogeneticity towards cells and the hemagglutinating capacity, which, as is known, are imparted to the molecule by the presence of the B oligomer.

In fact Bordetella strains according to the present invention which secreted only said oligomer (indicated with B in Table II) assayed *in vitro* for the presence of mitogenetic activity confirm said teachings of the art.

Although the role of activity *in vivo* is still nuclear, one can foresee that it should be minimal or absent, because in order to have an ascertainable mitogenic effect *in vitro*, high concentrations (0.3-1.0 µg/ml) are necessary. Such concentrations are only present at the site of vaccine inoculation.

According to the present invention, while not limiting it, the immunogenic properties of the PT-9K/129G mutant were tested *in vivo* as reported in the examples that follow. The results showed that said mutant is capable of inducing the formation of anti-PT antibodies with high antibody titers and that said antibodies were capable of neutralizing the PT toxic effect on CHO cells.

One can therefore conclude that the genetic manipulations performed for the construction of the mutagenized PT gene do not alter the typical immunogenic properties of the pertussis toxin and that, in distinction from what has been reported in the prior art, said properties are independent from the enzymatic activity of the PT S1 subunit.

The Bordetella strains and the enzymatically inactive PT mutants (with reduced or no toxicity) obtained according to the present invention, are therefore excellent candidates for the development of effective antipertussis vaccines.

According to the present invention, immunogenic formulations suitable as antipertussis vaccines may be prepared by adding said strains or the mutants produced by them to a pharmaceutically acceptable carrier selected from the ones generally used as vehicles for immunogenic materials in a patient. An example of such a carrier is saline solution. The antigen may be present in the carrier in solution or in suspension. Said formulations may also comprise an adjuvant to stimulate the immune response and therefore improve the vaccine effectiveness. Suitable adjuvants include, for instance, aluminium phosphate, aluminium hydroxide, interleukin-1 or 2 or their peptide fragments.

Immunogenic formulations suitable as antipertussis vaccines contain, generally, a final concentration of strains and of mutants produced by them selected in order to impart an effective immunity against pertussis. The vaccine, after formulation, may be introduced into a sterile container and kept at various temperatures, for instance 4°, 20° or 37° or lyophilized. To induce an effective immunity against pertussis, one or more doses of the conveniently formulated vaccine may be administered. Vaccines according to the present invention may be administered according to conventional methods. The treatment may consist of administering one dose or successive doses. Vaccines according to the present invention may comprise one or more antigens such as, for example, tetanus toxoid or diphtheria toxoid or other Bordetella antigens.

In order to assure the best formulations to be included in an antipertussis vaccine, PT mutants may be stabilized with formaldehyde in amounts, expressed in wt/vol, of between 0.035% and 0.420%, corresponding to a PT mutant/formaldehyde wt. ratio of between 0.300 and 0.025.

Formaldehyde, employed in such concentrations, beside allowing the mutant stabilization, incudes a reduction and/or disappearance of mitogenicity and of the hemagglutinating activity, depending on the employed concentration, without altering the immunological properties.

In distinction from what is described in the literature for the CRM197 of diphtheria toxin, in which the formaldehyde treatment of the molecule was necessary to obtain a protective immunity, we have surprisingly found that the PT mutants, both stabilized and non stabilized with formaldehyde, showed identical immunological activities (induction of neutralizing antibodies and protection against intracerebral infections by virulent B. pertussis).

Both formulations (whether containing the stabilized mutant or not) showed the same stability when kept at 20°C or 4°C, while at 37°C a higher stability is observed for the formaldehyde treated mutant.

Antipertussis vaccines according to the present invention show considerable advantages with respect to those of the prior art containing, as an active component, PT detoxified by means of chemical reagents. The PT mutants obtained by genetic manipulation according to the present invention showed in fact an irreversible toxicity alteration and an unaltered immunogeneticity.

The safety of the PT mutants according to the present invention was further confirmed by the evidence that *in vivo* treatment (mice and rats with 1500 µg/kg body weight, which is 1000 times the foreseen human dose) did not lead to any local or systemic toxic reaction.

In conclusion, Bordetella strains mutated according to the present invention, and, preferably, the mutated PT toxins produced by them, are, for their high immunogenecity and absence of toxicity, particularly suitable antigens for the development of synthetic cellular and acellular antipertussis vaccines having desired characteristics.

In accordance with the present invention, *Bordetella pertussis* (W28) PTL9/28G (PT-9K/129G), *Bordetella pertussis* PT28G (PT-129G) and *Bordetella parapertussis* PT126/28G (PT-26I/129G) were deposited at the American Type Culture Center on April 5, 1989, as ATCC 53894, ATCC 53892 and ATCC 53893.

### BRIEF DESCRIPTION OF THE DRAWINGS

Figure 1: schematic representation of the method employed for removing the pertussis toxin gene from the Bordetella strains' chromosomes and substitution with genes encoding mutated toxins or kanamycin.

Figure 2: the graph shows in the ordinates the optical density (O.D.) and the production of PT-129G obtained by cultivation of *B. pertussis* W28/PT129G (X), *B. bronchiseptica* 7865/PT-129G (■) and *B. parapertussis* P14/PT-129G (□) strains and in the abscissae the time in hours.

Figure 3: 15% polyacrylamide gel of PT wild type (A) toxin and of the purified mutants PT-9K (B), PT-129G (C), PT-26I/129G (D), PT-13L/129G (E) and PT-9K/129G (F).

Figure 4: the graph shows in the abscissae the dose of PT and of PT mutants expressed as µg/mouse and in the ordinates the number of leucocytes x 10⁶ ml⁻¹.

Figure 5: electrophoretic pattern of wild type PT (lanes 1, 4), of the PT-9K/129G non stabilized mutant (lanes 2, 5) and of the same mutant stabilized with formaldehyde (PTF-9K/129G) (lanes 3 and 6) at day 0 (lanes 1, 2, 3) and after 1 month at 37°C (lanes 4, 5, 6).

Figure 6: shows the PBMC mitogenic response to wild type PT and to the PT-9K/129G mutant. The PBMC utilized in this test did not show any significant antigen-specific response to heat inactivated PT. The standard deviations were lower than 15%.

Figure 7: shows the antibody titer (ELISA assay) and the neutralizing capacity of antibodies obtained in guinea pigs after 1 or 2 subcutaneous injections of PT or of different doses of PTF-9K/129G adsorbed on A1(OH)₃. The antibody antitoxin levels are expressed as values of maximum absorbance of undiluted sera (ABS-MAX). The neutralizing titers (NT) are expressed as the reciprocal of the highest serum dilution capable of inducing 100% inhibition of the agglutinating effect on CHO cells induced by 120pg PT assayed in triplicate.

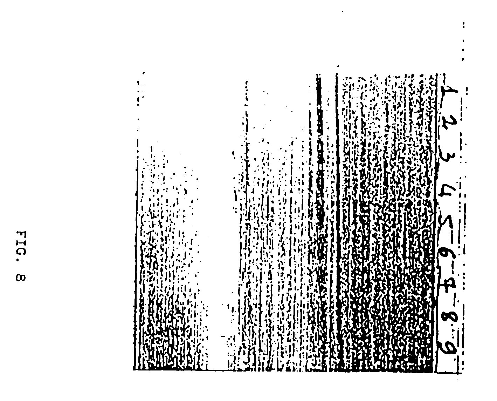

Figure 8: electrophoresis on SDS polyacrylamide gel of PT-9K/129G treated with different percent concentrations of formaldehyde (0.035, 0.042, 0.052, 0.070, 0.105, 0.140, 0.210 and 0.420%).

The following examples are illustrative and not limitative of the present invention.

### Example 1

### Construction of Bordetella (Δ tox) mutants free of the pertussis toxin gene

*Bordetella pertussis* strains BP165, BP Tohama and BPW28 (SCLAVO SpA), *Bordetella parapertussis* BP14 (SCLAVO SpA) and *Bordetella bronchiseptica* BP7865 (SCLAVO SpA) were made resistant to streptomycin (str) and to nalidixic acid (nal).

In practice, approximately 10¹⁰ bacteria of each strain were plated on Bordet-Gengou (BG) agar (DIFCO) medium supplemented with 15% defibrinated sterile blood containing 800 µg/ml str or 200 µg/ml nal and cultivated at 37°C for about 100 hours.

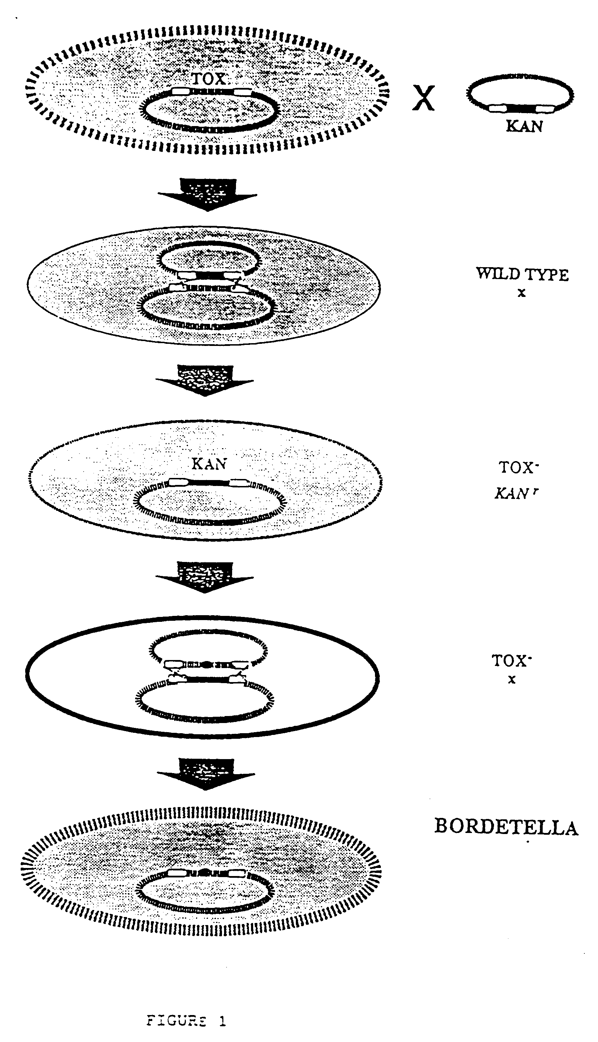

The spontaneous mutants grown on said plates were isolated and the gene encoding the pertussis toxin, contained in their chromosome was substituted with the kanaymcin structural gene, operating according to the scheme reported in Figure 1.

For this purpose, plasmid pRTP1 (Stibiz *et al*. Gene, vol. 50 1986, p. 133-140) was employed which does not replicate in Bordetella, but can be introduced into it by conjugation.

In practice, plasmid pRTP1 (10 µg) was digested with 50 units of restriction enzyme EcoRI according to the method suggested by the supplying firm (BRL). The plasmid DNA was then ligated in 10 µl of ligase buffer (66 mM Tris-HC1 pH 7.6, 1mM ATP, 10mM MgC12, 15 mM dithiothreitol) in the presence of 1 unit T4 DNA ligase, at 14°C for one night, with 0.2 µg DNA EcoRI fragment containing the structural gene encoding for resistance to Kanamycin (kan) (Pharmacia, Uppsala) comprised among the nucleotide sequences corresponding to regions 1-420 and 3626-4692 which flank the 1-420 and 3626-4692 which flank the structural pertussis toxin gene. Said fragment was obtained by digesting first plasmid DNA PT101 ATCC67854 with restriction enzyme BstEII, which cuts only at restriction sites in position 421 and 3625 and eliminates sequence 421-3625, and then making blunt-ends by means of the Klenow enzyme. Finally, fragment HincII containing the kan gene was ligated with the linearized plasmid DNA as reported supra in a ligase buffer in the presence of T4 DNA ligase. After approximately 18 hours at 14°C, the ligation mixture was employed to transform competent *E. coli* cells and the transformants were selected on LB agar medium with added 50 µg/ml ampicillin and 50 µg/ml kanamycin at 35°C for one night. Finally, from one of the positive clones, the plasmid having the expected characteristics was isolated and successively digested with EcoRI restriction enzyme. The DNA fragment containing the kanamycin gene is isolated on agarose gel as described by Maniatis *et al* (1983) "Methods in Enzymology".

Said EcoRI fragment is then ligated with pRTP1 plasmid previously digested with EcoRI and the resulting ligation mixture was employed to transform *E. coli* SM10 cells described by Simon R. *et al* (Biotechnol., vol. 1, p. 784-791 - 1983) made competent as described by Messing in "Methods in Enzymology" vol. 101, 20-78, 1983.

The transformants were selected on LB agar plates (DIFCO, Lab.) containing 50 µg/ml ampicillin and 50 µg/ml kanamycin, at 37°C for 24 hours.

From one of the positive clones, the plasmid denominated pRTP1-Δ PT-KAN having the expected characteristics was extracted. Successively *E. coli* SM10 cells transformed with said plasmid and cultivated on LB agar at 37°C for about 18 hours, were conjugated with the Bordetella str or nal resistant strains previously cultivated on BG medium for 48 hours. The conjugation was performed on BG medium with 10 mM MgC1₂ added at 37°C for 3-6 hours. The resulting colonies were then harvested and plated on BG medium containing 30 µg/ml nalidixic acid and 50 µg/ml kanamycin. The plates were kept at 37°C for the purpose of selecting the strains resistant to such antibiotics. After 3 days (*B. bronchiseptica*) and 5-6 days (*B. pertussis* and *B. parapertussis*) numerous single hemolytic colonies were observed, resistant to nal and kan, which contain in their chromosome the pRTP1-ΔPT-KAN plasmid integrated by homologous recombination with one of the regions flanking the pertussis toxin gene. In order to facilitate the recombination of also the second region, and hence the substitution of the PT chromosomal gene with the one of Kanamycin, the colonies were again plated on BG medium containing 400 µg/ml streptomycin. Operating as reported above, strains were selected which had lost the pRTP1 plasmid imparting a sensitivity to streptomycin which is dominant on the chromosomal Bordetella resistance.

Colonies of two types were thus obtained:
1) the ones resistant to str and nal and sensitive to kan in which complete plasmid loss and absence of recombination has taken place and
2) the ones resistant to str, nal and kan in which through double recombination (Δtox) the substitution of the kanamycin gene for the PT gene had taken place.

For the purpose of confirming such chromosomal substitution, strains Δ tox W28, Δ tox Tohama, Δ tox 165, Δ tox P14 and Δ tox 7865 were characterized, operating according to known techniques, by means of Southern blotting, ELISA assay and toxicity on CHO cells. In practice, the chromosomal DNA isolated from said Bordetella strains by the method of Marmur, J., J. Mol. Biol. (1961), 3: 208-216, was digested with suitable restriction enzymes, submitted to electrophoresis, transferred on nitrocellulose membranes and then hybridized employing as probes the 4696bp EcoRI fragment containing the PT gene, and the DNA fragment containing the kan gene radioactively labelled using the BRL nick-translation kit.

The hybridization reaction was performed operating according to the method of Southern, E. (1975), J. Mol. Biol., 98: 503-517.

The results showed the presence in the Bordetella Δ tox strains' chromosomal DNA of a DNA fragment with a molecular weight lower than the one of the PT gene which hybridized with both probes.

The Bordetella Δ tox strains were cultivated, at 37°C for 72 hours, in SS modified medium the composition of which, in grams/liter was as follows:
Sodium L-glutamate 10.7; L-proline 0.24; NaC1 2.5; KH₂PO₄ 0.5; KC1 0.2; MgC1₂ x 6H₂0.01; CaC1₂ 0.02; TRIS 6.1; L-cysteine 0.04*; FeSO₄ x 7H₂0 0.001*; niacin 0.004*; glutathione 0.10*; ascorbic acid 0.02*; resumin acids 10.0; 2,6-0-dimethyl beta cyclodextrin 1.0, pH 7.6

The medium is sterilized for 20 minutes while the components marked * were sterilized separately by filtration. At regular intervals, medium samples were taken and centrifuged at 12000 rpm for four minutes at 4°C. Successively, aliquots of the cell-free supernatants were assayed with the ELISA test and toxicity on CHO cells, in order to verify the presence of pertussis toxin and its toxicity.

The ELISA assay, performed as described by Wong, K.H., and Skelton, S.K., J. Clin. Microbiol., vol. 26, 1316-1320, 1988, showed that none of the Δ tox strains was capable of producing detectable amounts of PT.

Furthermore, the supernatants diluted 1/10, did not modify the CHO cells (Hewlett, E.L. *et al*., (1983), Infect. Immun, 40: 1198-1230). A nonspecific toxicity was observed utilizing the undiluted supernatant.

To verify whether said strains, although not producing PT, were still capable of imparting protection against virulent *B. pertussis*, intracerebral challenge tests were carried out according to the technique described in CODE OF FEDERAL REGULATION, potency test of pertussis vaccine, 21/Par7620. In practice, the Δ tox W28 and Tohama strains and the same wild type strains, generally employed for the preparation of antipertussis vaccine, were cultivated in 300 ml modified SS medium at 37°C up to an optical density of 0.7 measured at 590nm. The cultures were then centrifuged at 10000 rpm for ten minutes (Beckman J21 centrifuge with J10 rotor) and the cells, separated from the supernatants, were suspended again in 50 ml saline solution and kept at 56°C for 30 minutes. Successively, the resulting suspensions were suitable diluted as described in CODE OF FEDERAL REGULATION and utilized as conventional vaccines employing different doses. The results are reported in the following Table I:

**TABLE 1**

| dose ml/mouse* | survival to intracerebral challenge | | | |
|---|---|---|---|---|
| | Tohama | Tohama Δ tox | W28 | W28 Δtox |
| 0.04 | 15/16 | 14/16 | 16/16 | 16/16 |
| 0.008 | 13/16 | 11/16 | 16/16 | 13/16 |
| 0.0016 | 9/16 | 6/16 | 11/16 | 8/16 |
| 0.00032 | 2/16 | 2/16 | 4/16 | 2/16 |

As one can observe from the table, although a slight protection decrease for the Δ tox strains was observed, they still impart very good protection, and, therefore, appear to be particularly suitable as antipertussis vaccines. The * indicates the volume of cellular suspension.

### Example 2

### Construction of Bordetella mutants producing PT forms with altered toxicity

For the purpose of introducing mutated forms of the pertussis toxin gene in the chromosome of Δ tox strains obtained as reported in Example 1, the pRTP2 plasmid was modified by introducing in the BamHI site the gene encoding for resistance to gentamycin (Pharmacia, Uppsala) and the one encoding for resistance to tetracycline (Pharmacia, Uppsala). Cloning of said genes is performed employing the known recombinant DNA techniques described by Maniatis *et al*. These new vectors, designated respectively pRTPG1 and pRTPT1, were then employed for introducing into the Bordetella chromosome the mutated strains of pertussis toxin. In particular, said genes were obtained by deletion or substitution, by site-directed mutagenesis, in the gene encoding the PT contained in the PT101 ATCC 67854 plasmid, of nucleotide sequences by others which encode for different amino acids. More particularly, PT genes were constructed containing in the S1 nucleotide sequence the mutations reported in the first column of the following Table II.

After cloning the EcoRI fragments containing the above reported mutations in the pRTPG1 and pRTPT1 plasmids, were employed for transforming SM10 *E. coli* cells and the thus obtained transformants were conjugated with the Δ tox Bordetella strains. The colonies showing the integration of such plasmids in their chromosome were then selected on BG medium plates containing, respectively, 30 µg/ml nal and 20 µg/ml gentamycin or 30 µg/ml nal and 12.5 µg/ml tetracycline. All the thus selected colonies showed the plasmid integrated in their own chromosome. Successively, to the end of selecting for plasmid loss, the colonies obtained as reported were plated on BG medium containing 400 µg/ml streptomycine. The colonies capable of growing on said medium were then simultaneously cultivated on BG plates containing respectively:
a) nal 30 µg/ml, str 400 µg/ml and 50 µg/ml kanamycin;
b) nal 30 µg/ml, str 400 µg/ml.

The colonies obtained in a) were those which have lost the plasmid and are therefore the same as the original Δ tox colonies being still resistant to kanamycin.

The colonies grown on medium b), on the other hand, had lost the resistance to kanamycin, the gene of which was substituted by the mutated one.

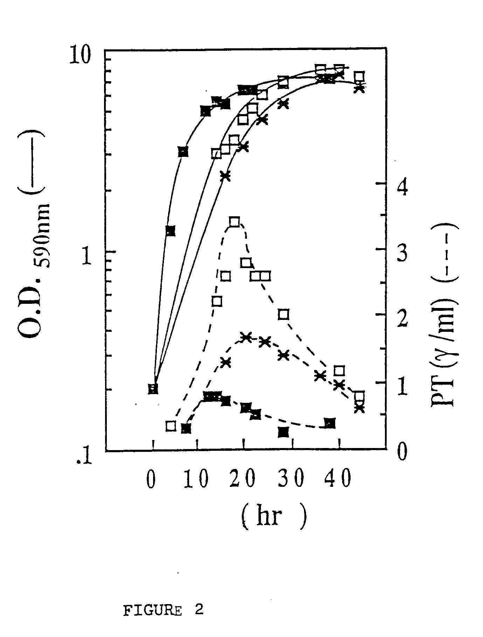

As an example of the capacity of the colonies obtained in b) to produce and secrete a PT mutant, the *B. pertussis* W28/PT-129G, and *B. bronchiseptica* 7865/PT129G strains are first expanded on BG plates and then cultivated in 15ml modified SS medium, at 37°C for 72 hours. The data for the PT mutant production, evaluated by monitoring with the ELISA assay, are reported in Figure 2, and show that:
- all the tested Bordetella strains produced the PT mutant and *B. parapertussis* produced a double amount with respect to other Bordetellas.

Data on production of PT mutants, obtained by cultivating as reported above *B. pertussis* W28 and BP165 and *B. parapertussis* P14 strains containing the PT gene comprising the mutations reported in the first column of Table II, are shown in the third column of the same table and indicate that:
- all the tested strains were capable of expressing and secreting the PT mutants;
- some of said PT mutants were produced in an amount comparable to the one obtained for the wild type PT (++++) and
- *B. parapertussis* P14 produced a double amount of PT mutants with respect to *B. pertussis*.

Some of said strains (indicated with B) secreted only the oligomer B of the pertussis toxin (constituted by subunits S2, S3, S4 and S5).

The results obtained after the intracerebral challenge tests showed, furthermore, that said Bordetella strains are suitable for the development of antipertussis cellular vaccines.

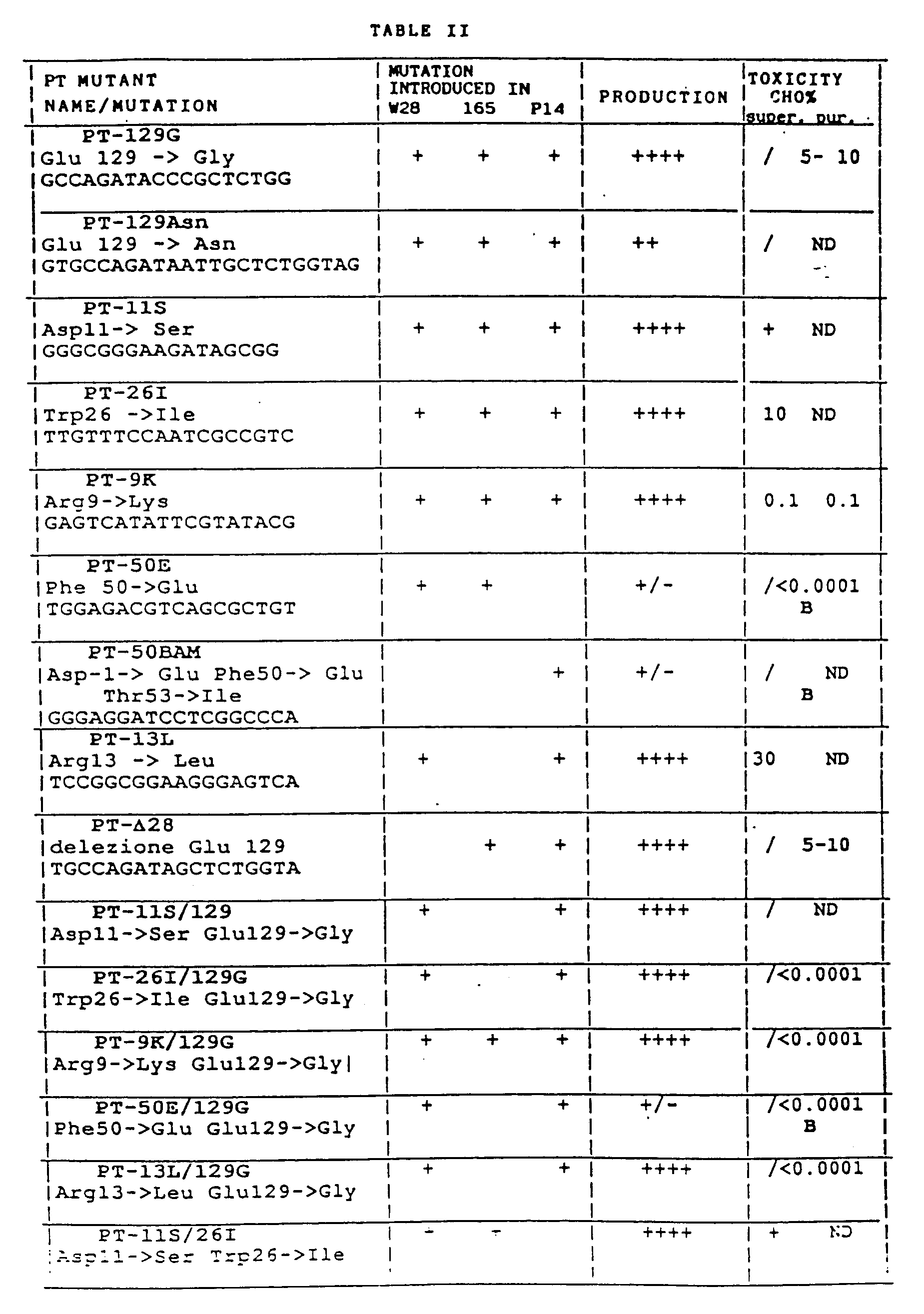

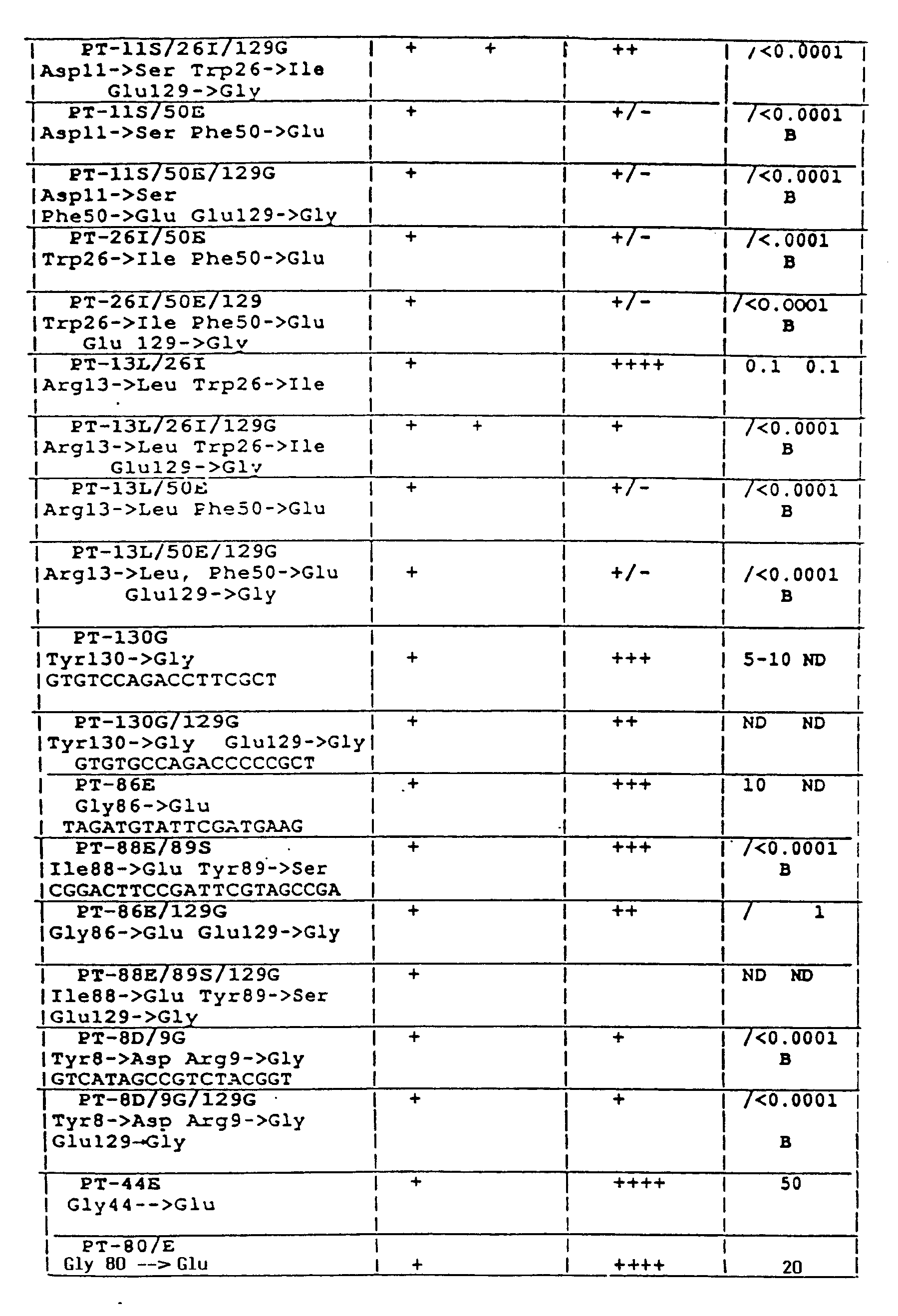

### Example 3

### A) Production and purification of PT mutants

The *B. parapertussis* P14/PT-129G, *B. pertussis* W28 9K/129G, *B. pertussis* W28 13L/129G and *B. pertussis* W28 26I/129G strains were cultivated in a Chemap fermentation vessel of 30 1 capacity containing 20 1 SS modified medium (pH 7.4), with an air flow of 0.1 v/v/m. The dissolved oxygen was kept at 20% varying the rotations per minute from a minimum of 200 to a maximum of 700. The temperature was controlled at 35°C and the pH was kept within neutrality values by using a solution containing 1.5 N glutamic acid, 1.5 N hydrochloric acid, and 0.15 N proline. After approximately 36-48 hours, that is when the cultures had reached an optical density measured at 590nm of 14-18, the cells were removed by centrifugation of the fermentation medium (Beckman JCF-7 centrifuge, 19000 tpm. 4°C for 30 minutes, with a flow of 600 ml/minute) and the mutated proteins were purified from the cell-free supernatant, filtered in sterile conditions (through a Durapore^{R} cartridge (Millipore) of 0.22 mµ) by absorption on Affi-Gel Blue and successive affinity chromatography on Sepharose-fetuin^{R} as described by Sekura R., D., *et al*. (J. Biol. Chem. 258: 14647-14561, 1983).

### B) Determination of the physicochemical properties of the purified PT mutants

The physicochemical properties of some PT mutants purified as reported above, were determined by gel electrophoresis on polyacrylamide sodium dodecyl sulphate (SDS) coloured with Coomassie blue operating as described by Laemmli, UK, (1970), Nature, 227, 680-685.

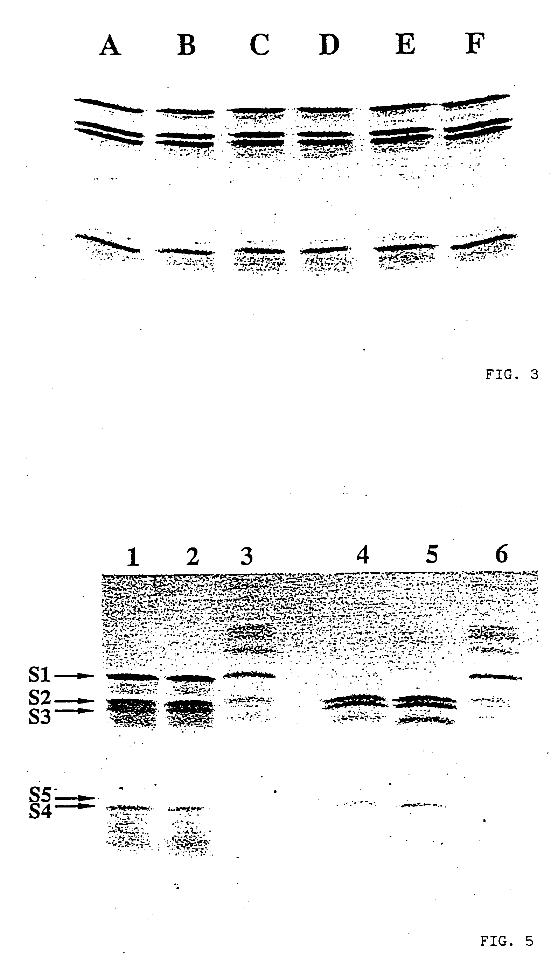

The results, reported in Figure 3, showed the absence of contaminant proteins and a pattern identical to the one of wild type PT.

A further control, performed to verify whether other contaminants were present, in particular the substances utilized in the fermentation process for the preparation of the mutants, confirmed:
1) the absence of dimethyl (2,6-0-)beta-cyclodextrin determined according to the method described by Beeley, J.G., (1985) "laboratory techniques in biochemistry and molecular biology", Burdon, R.H. and Van Knippennberg, P.H. (Edit.), Elsevier Vol. 16);
2) the absence of fetuin, of 69 KD protein and of filamentous hemagglutinin, which are the possible contaminants of acellular antipertussis vaccines, determined by Western blot analysis (Towbin, H.T. *et al*., (1976), PNAS, USA, 73:361-365) utilizing antibodies specific for said proteins;
3) the absence of thermonecrotic toxin, determined by the test performed on guinea pigs as described by Kume, K.T. *et al*., (1986), Infect. Immun., 52: 370-377); and
4) the absence of cyclodextrin, which is the major component of the culture medium, determined by thin layer chromatography. The PT mutants (80% yield) show a purity of 99%.

### Example 4

### In vitro characterization of PT mutants

### A) Toxicity on CHO cells

The test was performed utilizing the crude and purified supernatants of cultures of some Bordetella strains containing the mutations reported in the first column of Table II, diluted 1/10 in DMEM medium (Flow Lab., Mclean, Va).

The results reported in the preceding Table II showed a reduction in the toxicity of the pertussis toxin mutants with respect to wild type PT; the best results are obtained for mutants PT-26I/129G, PT-9K/129G and PT-13L/129G, for which absence of toxicity is observed.

Further, the nondetoxified PT-129G mutant shows, with respect to wild type pertussis toxin, a residual toxicity of 1.5-10%, while the same mutant detoxified with glutaraldehyde as described by Munoz, J.J. *et al*. (Infect. Immun. 32: 243-250, 1981) does not show any appreciable toxicity on CHO cells.

### B) Determination of the affinity constant by means of an RIA test

With this test, the affinity constant of some PT mutants for polyclonal antibodies (anti PT goat gamma-globulins, SCLAVO SpA) and monoclonal anti-S1 antibodies (1B7, described by H. Sato *et al*. (1984), Infect. Immun., 46:422-428) was determined.

In each well of a 96 well polystyrene flat bottom microplate (Dynatech Laboratories Inc., Alexandria, VA) 200 µl glycine buffer 5 mM pH 9.2 containing 10 µg/ml of antibodies were introduced. After one night at 4°C, the plates were saturated with 2.5% (weight/volume) of bovine serum albumin (BSA) in phosphate buffered saline (PBS) pH 8.0 and washed with 100 µl PBS containing 0.125 ml/1 Tween-20. The plates were then incubated with 10⁵ cpm (counts per minute) in each well of pertussis toxin labelled with ¹²⁵I, in the presence of different concentrations (0.01-0.025-0.05-0.1-0.25-0.5-1.0 µg/m) of PT and PT mutants. After three hours at room temperature (20-25°C), the plates were extensively washed with PBS and the incorporated radioactivity was measured in a gamma counter (Packard Inst., USA). Each sample was analysed twice. The pertussis toxin was labelled with radioactive iodine by the standard chloramine T method (BDH Chem., England) operating according to the instructions of the supplying firm.

The results, reported in the following Table III, showed that all the PT mutants maintained the recognized epitope of the monoclonal 1B7 antibody and that they are recognized as having high affinity from pertussis antitoxin goat gamma-globulins and therefore capable of neutralizing the PT toxin.

**TABLE III**

| AFFINITY CONSTANT (Ka(L/mol) | | |
|---|---|---|
| PT Mutants | MAb 1B7 | anti-PT goat immunoglobulins |
| PT | 3.5 x 10⁸ | 5.0 x 10¹⁰ |
| PT-129G | 2.1 x " | 1.7 x " |
| PT-9K | 8.9 x " | 1.0 x " |
| PT-13L/129G | 1.3 x " | 1.3 x " |
| PT-26I/129G | 5.5 x 10⁷ | 8.9 x 10⁹ |
| PT-9K/129G | 3.3 x 10⁸ | 1.2 x 10¹⁰ |

### Example 5

### In vivo characterization of PT mutants

The biological properties of some PT mutants and the possibility of their use in an antipertussis vaccine were tested by means of:

### A) Intracerebral challenge

The test was performed utilizing the standard cellular vaccine (control) and the PT-129G mutants, as such and detoxified with glutaraldehyde (PT-129G Det) and PT-26I/129G. The results are shown in Table IV.

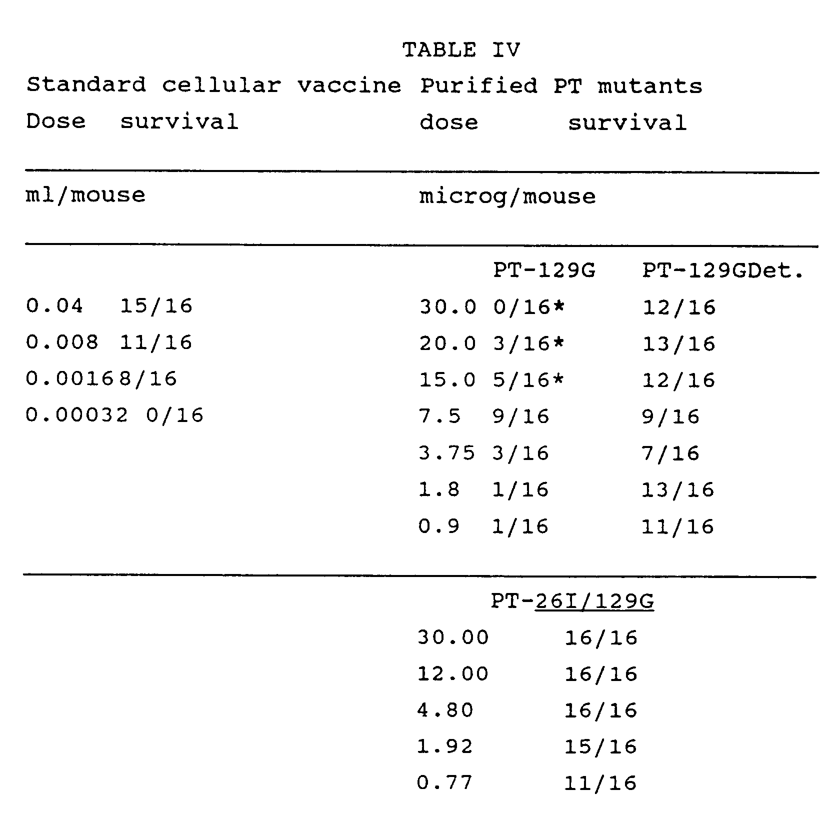

The low survival (*) obtained utilizing PT-129G is due to the mutant residual toxicity which is approximately 1-2% of the one of PT toxin.

### B) Leucocytosis

Groups of 4 female Balb/C mice of 7-8 weeks ago, weighing approximately 20g were treated by intravenous injection at day 0, with 0.2 ml physiological (saline) sterile apyrogenic solution as such (control) or containing:
- (0.004 - 0.02 - 0.04 - 0.1 - 0.5 and 1.0 µg/mouse) of PT.
- (0.1 - 0.5 - 2.5 - µg/mouse) of PT-13L;
- (0.1 - 0.5 - 2.5 - 12.5 - 25.0 and 50.0 µg/mouse) of PT-9K, PT-129G, PT-129G detoxified, PT-26I/129G, PT-13L/129G and PT-9K/129G.

After three days, the mice are bled and the number of total mononucleated cells (PBMC)/ml peripheral blood is counted individually in turk solution (0.01% gentian violet and 3% acetic acid). A portion of peripheral blood for each mouse, previously treated with red corpusclelysing solution, is then employed in a FACS (Fluorescence Activated Cell Sorter) to measure the percentage increase of lymphocytes and of polymorphonucleated cells in the mice treated with the different toxins, with respect to the controls.

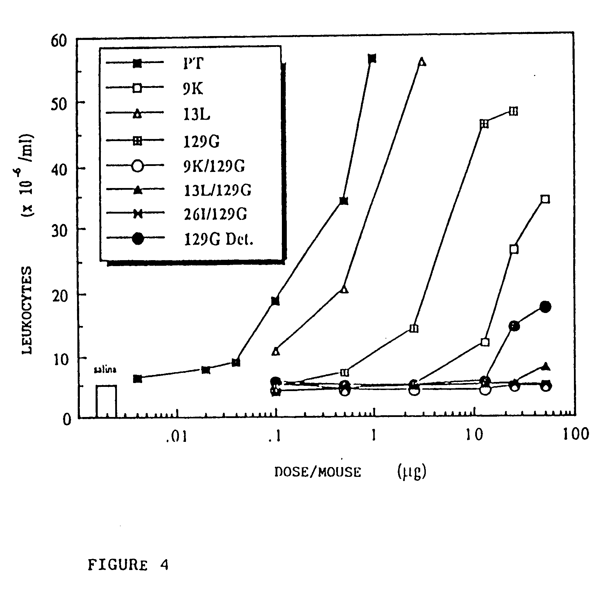

The results reported in Figure 4, showed, in general, a reduction of the PT mutant toxicity with respect to PT, which reached values lower than 0.01% for mutants PT-26I/129G, PT-9K/129G and PT-13L/129G.

The same test was performed on groups of Balb/C mice by intraperitoneal injection on day 0 of 0.5ml physiological sterile solution as such, or containing PT or the PT-9K/129G mutant in the same concentrations reported above.

Three days after the administration, blood samples were taken from the orbital plexus of the animals and tested as reported above. The results, expressed as an average +/-the standard deviation of leucocytes counts from 5 animals individually tested, are reported in Figure 4 and in Table V relative to PT and PT-9K/129G.

**TABLE V**

| Antigen | Dose microg/mouse | Leukocytosis (PBMC/mlx10⁻⁶) |
|---|---|---|
| Saline | -- | 5.23 +/- 0.51 |
| PT | 0.004 | 5.94 +/- 0.41 |
| | 0.20 | 10.33 +/- 0.82 |
| | 0.040 | 14.34 +/- 0.83 |
| | 0.100 | 17.73 +/- 1.12 |
| | 0.500 | 45.73 +/- 3.76 |
| | 1.000 | 55.19 +/- 6.62 |
| PT-9K/129G | 2.500 | 4.45 +/- 0.41 |
| | 12.500 | 4.39 +/- 0.32 |
| | 25.000 | 4.79 +/- 0.44 |
| | 50.000 | 4.71 +/- 0.35 |

### C) Histamine Sensitivity

Groups of 5 female Balb/C mice of 7-8 weeks ago, weighing approximately 20 g were intraperitoneally inoculated on day 0 with 0.5 ml physiological saline sterile apyrogenic solution as such (control), or containing different doses of PT, of PT-129G as such or detoxified with glutaraldehyde (PT_129G Det.), of PT-9K and PT-9K/129G. Six days after the administration, the mice were intraperitoneally inoculated with 0.5 ml physiological sterile apyrogenic solution containing 4 mg/mouse histamine dihydrochloride (Sigma Chemical Company, St. Louis, MO).

Deaths were registered 24 hour after the histamine administration.

The results are reported in the following Tables VI and VII

**TABLE VI**

| DAY 0 | DAY +6 | DEAD/TOTAL |
|---|---|---|
| dose/mouse 0.5ml/i.p. | dose/mouse 0.5ml/i.p. histamine (4 mg) | |
| saline | " | 0/5 |
| PT 0.050 x10⁻³ µg | " | 0/5 |
| PT 0.500 x10⁻³ µg | " | 0/5 |
| PT 5.000 x10⁻³ µg | " | 0/5 |
| 50.000 x10⁻³ µg | " | 0/5 |
| PT 100.000 x10⁻³ µg | " | 0/5 |
| PT 500.000 x10⁻³ µg | " | 0/5 |
| PT 1.000.000 x10⁻³ µg | " | 3/5 |
| PT-129G 0.0005 µg | " | 0/5 |
| PT-129G 0.0050 µg | " | 0/5 |
| PT-129G 0.0500 µg | " | 0/5 |
| PT-129G 0.5000 µg | " | 0/5 |
| PT-129G 5.0000 µg | " | 0/5 |
| PT-129G 50.0000 µg | " | 5/5 |
| PT-129G Det 0.0005 µg | " | 0/5 |
| PT-129G Det 0.0050 µg | " | 0/5 |
| PT-129G Det 0.0500 µg | " | 0/5 |
| PT-129G Det 0.5000 µg | " | 0/5 |
| PT-129G Det 5.0000 µg | " | 0/5 |
| PT-129G Det 50.0000 µg | " | 0/5 |

**TABLE VII**

| DAY 0 | DAY +6 | DEAD/TOTAL |
|---|---|---|
| Dose/mouse 0.5 ml i.p. | dose/mouse 0.5 ml i.p. histamine (4 mg) | |
| saline | " | 0/5 |
| PT 0.010 µg | " | 0/5 |
| PT 0.050 µg | " | 0/5 |
| PT 0.100 µg | " | 0/5 |
| PT 0.500 µg | " | 1/5 |
| PT 1.000 µg | " | 4/5 |
| PT 5.000 µg | " | 5/5 |
| PT-9K 0.100 µg | " | 0/5 |
| PT-9K 0.500 µg | " | 0/5 |
| PT-9K 1.000 µg | " | 0/5 |
| 5.000 µg | " | 0/5 |
| PT-9K 50.000 µg | " | 5/5 |
| PT-9K/129G 0.100 µg | " | 0/5 |
| PT-9K/129G 0.500 µg | " | 0/5 |
| PT-9K/129G 1.000 µg | " | 0/5 |
| PT-9K/129G 2.500 µg | " | 0/5 |
| PT-9K/129G 5.000 µg | " | 0/5 |
| PT-9K/129G 7.500 µg | " | 0/5 |
| PT-9K/129G 12.500 µg | " | 0/5 |
| PT-9K/129G 25.000 µg | " | 0/5 |
| PT-9K/129G 50.000 µg | " | 0/5 |

### D) Anaphylaxis

Potentiation of anaphylactic sensitivity was determined according to the method described by Steinman L. *et al*., (1985), PNAS USA, 82: 8733-8736.

Groups of five female Balb/C mice of 7-8 weeks ago (H-2^{d}) weighing approximately 20 g, were intraperitoneally inoculated on days -1, +1 and +6 with 0.2 ml physiological saline sterile apyrogenic solution as such (control), or containing bovine serum albumin (BSA) (Sigma Chemical Company, St. Louis, MO). The same groups of mice were then intravenously inoculated on days 0 and +2 with 0.4 ml apyrogenic sterile saline solution as such, or containing 40, 100 and 500 ng/mouse of PT, 100, 500 and 2500 ng/mouse PT-129G as such or detoxified with glutaraldehyde (PT-129G Det.) or 500, 2500 and 7500 ng/mouse of PT-9K/129G. Deaths were registered two hours after the last BSA administration.

The results are reported in Table VIII.

**TABLE VIII**

| DAY -1 | DAY 0 | DAY +1 | DAY +2 | DAY +6 | dead / total |
|---|---|---|---|---|---|
| Dose/mouse 0,2 ml i.p | Dose/mouse 0,4 ml i.v. | Dose/mouse 0,2 ml i.p. | Dose/mouse 0,4 ml i.v. | Dose/mouse 0,2 ml i.p. | |
| Saline | Saline | Saline | Saline | Saline | 0/5 |
| BSA 1 mg | Saline | BSA 1 mg | Saline | BSA1mg | 0/5 |

| | PT | | PT | | |
|---|---|---|---|---|---|
| Saline | 40 ng | Saline | 40 ng | Saline | 0/5 |
| BSA 1 mg | 40 ng | BSA 1 mg | 40 ng | BSA 1 mg | 4/5 |
| Saline | 100 ng | Saline | 100 ng | Saline | 0/5 |
| BSA 1 mg | 100 ng | BSA 1 mg | 100 ng | BSA 1 mg | 4/5 |
| Saline | 500 ng | Saline | 500 ng | Saline | 0/5 |
| BSA 1 mg | 500 ng | BSA 1 mg | 500 ng | BSA 1 mg | 5/5 |

| | PT-129G | | PT-129G | | |
|---|---|---|---|---|---|
| Saline | 100 ng | Saline | 100 ng | Saline | 0/5 |
| BSA 1 mg | 100 ng | BSA 1 mg | 100 ng | BSA 1 mg | 0/5 |
| Saline | 500 ng | Saline | 500 ng | Saline | 0/5 |
| BSA 1 mg | 500 ng | BSA 1 mg | 500 ng | BSA 1 mg | 1/5 |
| Saline | 2.500 ng | Saline | 2.500 ng | Saline | 0/5 |
| BSA 1 mg | 2.500 ng | BSA 1 mg | 2.500 ng | BSA 1 mg | 3/5 |

| | PT-129G Det | | PT-129G Det | | |
|---|---|---|---|---|---|
| Saline | 100 ng | Saline | 100 ng | Saline | 0/5 |
| BSA 1 mg | 100 ng | BSA 1 mg | 100 ng | BSA 1 mg | 0/5 |
| Saline | 500 ng | Saline | 500 ng | Saline | 0/5 |
| BSA 1 mg | 500 ng | BSA 1 mg | 500 ng | BSA 1 mg | 0/5 |
| Saline | 2.500 ng | Saline | 2.500 ng | Saline | 0/5 |
| BSA 1 mg | 2.500 ng | BSA 1 mg | 2.500 ng | BSA 1 mg | 0/5 |

| | PT-9K/129G | | PT-9K/129G | | |
|---|---|---|---|---|---|
| Saline | 500 ng | Saline | 500 ng | Saline | 0/5 |
| BSA 1 mg | 500 ng | BSA 1 mg | 500 ng | BSA 1 mg | 0/5 |
| Saline | 2.500 ng | Saline | 2.500 ng | Saline | 0/5 |
| BSA 1 mg | 2.500 ng | BSA 1 mg | 2.500 ng | BSA 1 mg | 0/5 |
| Saline | 7.500 ng | Saline | 7.500 ng | Saline | 0/5 |
| BSA 1 mg | 7.500 ng | BSA 1 mg | 7.500 ng | BSA 1 mg | 0/5 |

### E) IAP Islet Activating Protein

The activation of pancreatic islets by PT or by PT-9K/129G was determined as described by Kreeftenberg, J.G. *et al*. (1984), J. Biol. Stand., 12:151-157.

Groups of 5 female Balb/C mice of 5-7 week age, weighing approximately 20g were intraperitoneally inoculated with 0.2 ml apyrogenic sterile saline solution (control), or containing 25 µg/ml PT9K/129G or 1 µg/ml PT. After 4 days the insulin levels in the mice serum expressed as mU/1 were determined.

The results showed, as expected, a significant increase of insulin secretion (19.6 mU/1) induced by PT, while the values of the secretion induced by the PT9K/129G mutants (5 mU/1) were comparable to the ones obtained with the control (8 mU/1).

### Example 6

### Formaldehyde treatment of PT-9K/129G mutant

### A) Study of the effect of the treatment on the mitogenicity, hemagglutinating activity and affinity constant of mutant PT-9K/129G

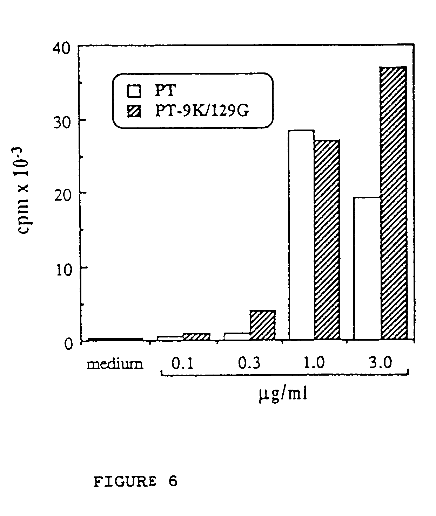

Mutant PT-9K/129G purified as reported in Example 3, was dialysed against PBS, pH 7.4, containing 0.025 lysine (Ajinomoto, Japan) and 0.01% merthiolate (ELANCO, USA), for 24 hours at 4°C and then suspended again in PBS. After determination of the protein contents (Lowry, O.H. *et al*., (1951), J. Biol. Chem., 193:265-275), aliquots of the mixture were supplemented with different concentrations (0.035% to 0.420% w/v) of formaldehyde (4% solution in PBS, pH 7.4) so as to obtain a final ratio of mutant to formaldehyde of between 0.300 and 0.025 (weight/weight). The resulting mixtures were incubated at 37°C for 48 hours, in the absence and in the presence of 0.025 M lysine, and then repeatedly dialysed against PBS. The mixtures were then tested to determine their free formaldehyde contents which are found to be lower than 0.01% (weight/volume). Furthermore, the mixtures, analysed on SDS-PAGE, showed the same electrophoretic pattern as PT and the presence of some extra bands, one of which migrates with subunits S2 and S3, and the other, with higher molecular weight, with subunit S1 (Figure 5, lane 3).

The mitogenic activity of the PT-9K/129G mutant treated with different concentrations of formaldehyde (PTF-9K/129G) was then determined by the response of human peripheral blood monoculeated cells (PBMC) isolated from normal adults and compared with that of PT and of the same mutant as such (Figure 6). In practice, the PBMC cells were plated in wells of 96 flat bottom well microplates (Costar, Cambridge, MA) at a concentration of 10⁵/well in 0.2 ml of RPMI 1640 (Gibco Laboratories, Paisley) supplemented with 2 mM L-glutamine, 1% non-essential amino acids, 10x10⁻⁵ M 2-mercaptoethanol and 10% human serum albumin PT and the PTF-9K/129G and PT-9K/129G mutants were then added in each well at a concentration of 0.1, 0.3, 1.0 and 3.0 and 6.0 µg/ml. After 96 hours of incubation at 37°C, into each well 1 microCi ³H thymidine (sp. act. 185 GBq/mmole; Amersham International, Amersham UK) was added. After 16 hours at room temperature, the cells were collected on glass wool filters with a cell collector (Skatron, Lier, Norway) and the incorporated radioactivity determined by liquid scintillation. The results reported in Figure 6 (PT, PT-9K/129G) and Table IX (PT-9K/129G and PTF-9K/129G) showed that:
1) the PT-9K/129G mutant maintained mitogenic activity against human T-lymphocytes comparable to that of PT protein. This is in accordance with the observation that said mitogenic activity is due to the presence of B oligomer, and
2) that increasing formaldehyde concentrations reduced, to disappearance, the mitogenic activity.

The hemagglutinating activity of mutant PTF-9K/129G was determined as described by Sato *et al*., (1983) Infect. Immun., 41:313-320, utilizing as target cells chicken red blood cells fixed with glutaraldehyde. The results reported in Table IX indicate that the treatment with increasing formaldehyde doses progressively reduced the hemagglutinating activity of the mutant.

The affinity constant was determined as described in the preceding Example 4. The results are reported in Table IX.

**TABLE IX**

| FORMALDEHYDE (dose) | | MITOGENICITY^{a} (µg/ml) | | | | HEMAGGLUTINATION^{b} (µg/well) | AFFINITY^{c} [Ka(L/Mol)] | |
|---|---|---|---|---|---|---|---|---|
| (%)^{d} | PT/F^{e} | 6 | 3 | 1 | 0.3 | | gamma-globulins | mAb(1B7) |
| - | - | 52.0 | 35.0 | 23.0 | 12.0 | 0.5-1 | 1.15x10⁹ | 5.54x10⁷ |
| 0.035 | 0.300 | 51.4 | 51.2 | 43.6 | 15.4 | 4 | 1.61x10⁹ | 7.4 x10⁷ |
| 0.042 | 0.250 | 45.8 | 37.0 | 30.1 | 10.1 | 4 | 1.67x10⁹ | 5.04x10⁷ |
| 0.052 | 0.200 | 15.6 | 48.3 | 29.6 | 10.7 | 4 | 8.25x10⁹ | - |
| 0.070 | 0.150 | 49.5 | 42.1 | 11.8 | 2.1 | 4 | N.D. | - |
| 0.105 | 0.100 | 33.1 | 19.8 | 4.7 | 0.9 | 9 | 1.85x10⁸ | - |
| 0.140 | 0.075 | 17.4 | 13.5 | 2.6 | 0.6 | >10 | 1.03x10⁸ | - |
| 0.210 | 0.050 | 12.4 | 11.3 | 2.0 | 0.3 | >10 | 5.60x10⁷ | - |
| 0.420 | 0.025 | 3.3 | 1.5 | 0.5 | 0.6 | >10 | 6.75x10⁷ | - |

| | | | | | | | | |
|---|---|---|---|---|---|---|---|---|
| where: "a" are the results expressed as average of counts per minute (cpmx10⁻³) for each culture tested in duplicate; | | | | | | | | |
| "b" are the results expressed as the protein dose which caused complete agglutination of the chicken red blood cells fixed with glutaraldehyde; | | | | | | | | |
| "c" is the affinity constant as determined by RIA test; | | | | | | | | |
| "d" is the percentage (weight/volume) formaldehyde in the sample as reported; and | | | | | | | | |
| "e" indicates the mutant/formaldehyde ratio (weight/weight) in the sample. | | | | | | | | |

### B) Study of the formaldehyde treatment on the PT-9K/129G stability

PT, the mutant PT-9K/129G as such, and the same mutant treated with 0.035% (W/v) formaldehyde (PTF-9K/129G) were kept at 4°, 20° and 37°C.

Then, the proteins were tested after 120 days (4°C and 20°C) and 30 days (37°C) to determine the electrophoretic profile and the affinity constant against polyclonal (anti-PT gamma globulins) (A) antibodies and monoclonal (1B7) (B) antibodies.

The results (Table X and Figure 5) indicate that the molecules kept at 4°C and 20°C for a period of 120 days did not undergo any variation of their electrophoretic pattern or their affinity constant.

The same molecules, kept at 37°C for thirty days, showed instead a progressive decrease of the intensity of the band corresponding to the S1 subunit for PT and the PT-9K/129G mutant (Figure 5, lanes 4 and 5), which was not observed for the PTF-9K/129G mutant (Figure 5, lane 6).

**TABLE X**

| | days | Conservation temperature | Affinity |
|---|---|---|---|
| PT (A) | 0 | 4°C | 2.0x10¹⁰^{a} |
| PT (B) | 0 | 4°C | 2.4x10⁸ |
| PT-9K/129G (A) | 0 | 4°C | 9.8x10⁹ |
| PT-9K/129G (B) | 0 | 4°C | 6.1x10⁸ |
| PTF-9K/129G (A) | 0 | 4°C | 1.7x10¹⁰ |
| PTF-9K/129G (B) | 0 | 4°C | 3.2x10⁸ |
| PT (A) | 120 | 4°C | N.D.^{b} |
| PT (B) | 120 | 4°C | N.D. |
| PT-9K/129G (A) | 120 | 4°C | 9.5x10⁹ |
| PT-9K/129G (B) | 120 | 4°C | 5.7x10⁸ |
| PTF-9K/129G (A) | 120 | 4°C | 1.4x10¹⁰ |
| PTF-9K/129G (B) | 120 | 4°C | 6.2x10⁸ |
| PT (A) | 120 | 20°C | N.D. |
| PT (B) | 120 | 20°C | N.D. |
| PT-9K/129G (A) | 120 | 20°C | 3.1x10¹⁰ |
| PT-9K/129G (B) | 120 | 20°C | 2.9x10⁸ |
| PTF-9K/129G (A) | 120 | 20°C | 1.5x10¹⁰ |
| PTF-9K/129G (B) | 120 | 20°C | 2.1x10⁸ |

| | | | |
|---|---|---|---|
| where: "a" are the data, evaluated by means of non-linear regression analysis, which are expressed as Ka(L/Mol) and represent the geometric average of the value obtained for a sample tested in triplicate. Standard deviation values are never higher than 15%. | | | |
| "b" N.D. = Not determined. | | | |

### C) Analysis of the amino acid composition

The analysis of the amino acid residues of mutant PT-9K/129G is performed, before and after treatment with 0.035% formaldehyde, as described by Spackman D.H. *et al*., (1958) Anal. Chem., 30:1190-1206. The acid hydrolysis of PT mutants was performed in 6N HC1 at 110°C for 24 hours in vials sealed under vacuum. The amino acid analysis was then performed employing an amino acid analysis apparatus (Kontron, Zurich, Switzerland).

During acid hydrolysis tryptophan was destroyed and therefore it was not possible to determine it. Further, because of the deamidation during the acid hydrolysis, asparagine and glutamine were transformed respectively into aspartic acid and glutamic acid. In Table XI the values corresponding to the sum of asparagine + aspartic acid (Asx) and glutamine + glutamic acid (Glx) are reported.

**TABLE XI**

| Amino Acids | PT | PT-9K/129G | PFT-9K/129G |
|---|---|---|---|
| Asx | 65 | 61.2 | 67.0 |
| Thr | 70 | 70.5 | 65.6 |
| Ser | 67 | 70.1 | 61.4 |
| Glx | 82 | 60.1 | 93.5 |
| Pro | 55 | N.D.^{b} | N.D. |
| Gly | 80 | 81.3 | 85.5 |
| Ala | 87 | 79.8 | 90.0 |
| Cys | 26 | N.D. | N.D. |
| Val | 67 | 72.9 | 67.9 |
| Met | 29 | 28.8 | 26.7 |
| Ile | 40 | 40.0 | 38.0 |
| Leu | 74 | 75.8 | 77.3 |
| Tyr | 62 | 63.5 | 61.2 |
| Phe | 32 | 30.5 | 31.0 |
| Lys | 32 | 39.3 | 164.9^{c} |
| His | 16 | 16.7 | 14.6 |
| Arg | 62 | 63.5 | 65.0 |
| Trp | 6 | N.D. | N.D. |

| | | | |
|---|---|---|---|
| where: "a" are the theoretical values deduced from the primary protein structure expressed as amino acid/protein ratio; | | | |
| "b" N.D. = not determined | | | |
| "c" the underlined value shows the lysine increment after formaldehyde treatment in the presence 0.025 M lysine. | | | |

### Example 7

### Toxicity of the PTE-9K/129G mutant on CHO cells

1 x 10⁴ CHO cells were incubated for 48 hours with different PTF-9K/129G doses (between 0.01 and 5 µg/ml) and PT doses (between 0.3 pg and 90 ng/ml). Then the minimum dose which was capable of causing the morphological change of the cells was determined.

The results showed that the PTF-9K/129G mutant was devoid of toxicity at the maximum tested dose (5 µg/ml) and was at least 10⁶ times less toxic than PT (5 pg/ml).

### Example 8

### In vivo characterization of the biological properties of the PTF-9K/129G mutant

### A) Anaphylaxis potentiation

The induction of anaphylactic sensitivity was determined according to the method described by Steinman, L. *et al*. (1985) PNAS USA, 82: 8733-8736.

Groups of 5 female Balb/C mice of 5-7 weeks, weighing approximately 20 g, were intraperitoneally inoculated, on days -1, +1 and +6, with 0.2 ml physiological sterile apyrogenic saline solution as such, or containing 1 mg/mouse BSA (Sigma Chemical Company, St. Louis, MO). The same groups of mice were then treated intravenously on days 0 and +2 with 0.2 ml apyrogenic sterile saline as such, or containing 0.04, 0.1 and 0.5 µg/mouse of PT or 2.5 and 7.5 µg/mouse PTF-9K/129G. Deaths were registered 2 hours after the last BSA administration. The results are reported in Table XII.

### B) Histamine sensitivity

Groups of female Balb/C mice of 5-7 weeks, weighing approximately 20 g were inoculated intraperitoneally on day 0 with 0.5 ml saline apyrogenic sterile physiological solution as such, or containing 0.004, 0.02, 0.04, 0.1, 0.5 and 1.0 µg PT or 2.5, 12.5, 25.0, 50.0 µg PTF-9K/129G.

At day +6 the mice were intraperitoneally inoculated with 0.5 ml apyrogenic sterile saline solution containing 4 mg histamine dihydrochloride.

Deaths were registered 24 hours after histamine administration. The results are reported in table XII.

### C) Leukocytosis

Groups of 5 female Balb/C mice of 5-7 weeks, weighing approximately 20 g were intraperitoneally inoculated on day 0 with 0.5 ml apyrogenic sterile solution as such, or containing 0.004, 0.02, 0.04, 0.1, 0.5 and 1.0 µg PT or 2.5, 12.5, 25.0 and 50.0 µg PTF-9K/129G.

On the third day after the administration, blood samples were taken from the orbital plexus of the animals and tested to determine leukocytosis. The results, expressed as average +/- the standard deviation of the leucocyte counts from 5 animals individually tested, are reported in Table XII.

**TABLE XII**

| | Dose µg/mouse | Leucoc. (PBMC/mlx10⁶) | Histam. D/T^{b} | Anaph. D/T^{b} |
|---|---|---|---|---|
| Saline | - | 5.23 +/- 0.51 | 0/5 | 0/5 |
| PT | 0.004 | 5.94 +/- 0.41 | 0/5 | N.D.* |
| | 0.020 | 10.33 +/- 0.82 | 0/5 | N.D. |
| | 0.040 | 14.34 +/- 0.83 | 0/5 | 4/5 |
| | 0.100 | 17.73 +/- 1.12 | 1/5 | 4/5 |
| | 0.500 | 45.73 +/- 3.76 | 5/5 | 5/5 |
| | 1.000 | 55.19 +/- 6.62 | 5/5 | N.D. |
| PTF-9K/129G | 2.500 | 4.45 +/- 0.41 | 0/5 | 0/5 |
| | 7.500 | N.D. | N.D. | 0/5 |
| | 12.500 | 4.39 +/- 0.32 | 0/5 | N.D. |
| | 25.000 | 4.79 +/- 0.44 | 0/5 | N.D. |
| | 50.000 | 4.71 +/- 0.35 | 0/5 | N.D. |

| | | | | |
|---|---|---|---|---|
| * N.D. = not determined | | | | |
| (b) = equal deaths on a total of 5 mice/group | | | | |

As can be seen from the table, the intraperitoneally inoculated mice develop a dose-dependent leukocytosis after three days. The increase of the peripheral blood mononucleated cells (BPMC) was statistically significant with 0.020 µg purified PT toxin, while the PTF-9K/129G was completely incapable of promoting leukocytosis at a dose of 50 µg/mouse.

Said mutant, intraperitoneally inoculated at the same doses as above, did not induce lethal effects in the mouse following the histamine administration. PT, on the other hand, inoculated at a dose of 0.5 µg/mouse caused 100% lethality.

The capacity of the pertussis toxin to potentiate the BSA anaphylaxis, a phenomenon which was associated with the encephalopathies cause by the cellular antipertussis vaccine, was absent for the PTF-9K/129G mutant.

Anaphylaxis potentiation, which caused death in 80% of the mice inoculated with 0.040 µg PT, was not observed in mice treated with 7.5 µg PTF-9K/129G.

### D) Acute Toxicity

The intraperitoneal or subcutaneous study of toxicity is performed according to the directions of OMS (WHO Tech. Rep. Ser. (1979), 638: 60-80).

Groups of 5 mice and 5 rats were intraperitoneally and subcutaneously inoculated with the PT-9K/129G and PTF-9K/129G mutants (1500 µg/kg of body weight). The animals were then kept under control for 14 days, during which no weight variations or other symptoms were registered which would indicate a local or systemic reaction.

### Example 9

### Development of the Antipertussis Acellular Vaccine

### A) Analysis of PTF-9K/129G Mutant Immunogenicity

For the purpose of investigating the PTF-9K/129G mutant immunogenicity, groups of 6 guinea pigs of 4 weeks age, weighing 350 g, were subcutaneously inoculated with 0.5 ml physiological apyrogenic sterile saline solution containing 0.001 mg sodium-ethyl-mercury-thiosalicylate and 3, 10, 25 and 50 µg PTF-9K/129G absorbed on A1(OH)₃ (1mg/ml) and 0.75 ml (corresponding to 1.5 human dose) of classical trivalent DPT vaccine (antidiphtheric, antipertussis and antitetanus) (SCLAVO, S.p.A.) in which the pertussis component consisted of killed *B. pertussis*.

After 4 weeks from the first inoculation, blood samples were taken and the animals were subcutaneously inoculated again with the same dose of PTF-9K/129G and DPT vaccine. After 2 weeks from the second inoculation, further blood samples were taken. The sera obtained from the samples are then tested with the ELISA assay to determine the antibody and anti-PT titer, and the CHO test to determine the PT neutralizing capacity of the antibodies. The ELISA assay, which was a modified version of the one described by Engvall and Perlmann (J. Immunol. 109: 129-135, (1972)) was performed as follows:

In each well of a polystyrene flat bottom micro plate (Dynatech Laboratories Inc., Alexandria, VA), 200 µl PBS, pH 7.4 were introduced containing 1 µg purified PT (antigen). The antigen adsorption on the solid phase was performed in a humidified chamber at 37°C for 3 hours and then at 4°C for one night. After minimization of the non-specific adsorption of the serum proteins on the plastic material with 1% BSA in PBS, the serum samples obtained from the guinea pigs and serially diluted down to 1:9120 with PBS with added 0.05% Tween-20, were introduced into each microplate well and incubated for 2 hours at 37°C. At the end, goat anti-guinea pig IgG antibodies conjugated with alkaline phosphatase (Miles, Yeda, Israel), diluted 1:3000 in PBS 0.05% Tween-20, were introduced in each well and the plates were incubated at 37°C for 2 hours. 100 µl volumes were utilized in all the steps and for washing the plates between incubations. The washings were carried out three times using PBS containing 0.05% Tween-20 and 0.02% NaN₃. The colorimetric reaction, which developed at room temperature in 30 minutes after adding the specific substrate (p-nitrophenyl phosphate, 1 mg/ml) was read at 405 nm in a Titertek Multiskan (Flow Laboratories, McLean, VA).

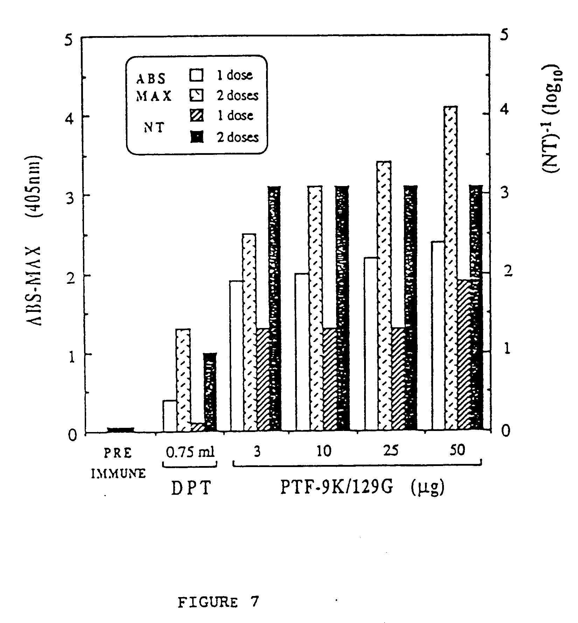

Controls for each plate included wells with serum samples free of antigen and viceversa. The antibody titers were evaluated, reporting in a graph (abscissae) the dilutions of the serum tested in duplicate vs. the average of the respective absorbences (ordinates). The intersection between the flex point and the absorbance axis represented the value of the undiluted serum absorbance (ABS-MAX). The ABS-MAX values obtained for each group of animals immunized with DPT and different doses of PTF-9K/129G were compared, after each sampling, with the respective pre-immune sera (Figure 7). The ABS-MAX increase was considered statistically significant when it was at least 4 times higher than the value obtained for a pre-vaccination serum.

As can be observed from Figure 7, the undiluted sera taken after 4 weeks after the first inoculation showed absorbance values (ASS-MAX, 1 dose) at 405 nm, which were from 32 to 40 times higher than the one determined before immunization. A further 50% increase was observed for the sera taken after two weeks from the second inoculation (ASS-MAX, 2 doses).

The increase of the antibody titer observed after PTF-9K/129G immunization correlated with the neutralizing activity determined in the CHO test. In fact, sera obtained after a single injection of 3, 10, 25 and 50 µg PTF-9K/129G was capable of neutralizing the agglutinating effect on CHO cells induced by 120 pg PT at dilutions of 1/20, 1/20, 1/20 and 1/80 respectively. The capacity for neutralizing the toxin increases considerably after the second immunization. In fact, antibodies obtained from animals immunized with 3 µg PTF-9K/129G were capable of neutralizing the toxin activity at a dilution of 1/1.280 (Figure 7).

### B) Analysis of the Potency of a Cellular Antipertussis Vaccine in Mice

Groups of 16 CD1 (Charles River, Calco, Italy) male mice of 3-4 weeks, weighing approximately 16 g, were intraperitoneally inoculated in accordance with the OMS norms with 0.5 ml sterile saline solution containing 0.24, 1.20, 1.92, 4.80, 12.00 and 30,00 µg fluid (non absorbed) PT-9K/129G and 0.24, 1.20, 6.00 and 30.00 µg of the same mutant stabilized with 0.035% formaldehyde (PTF-9K/129G) absorbed on A1(OH)₃ (1mg/ml). As a positive control, groups of mice were intraperitoneally inoculated with 0.00032, 0.001, 0.008 and 0.04 ml standard antipertussis cellular vaccine (National Institute of Health, Bethesda, MD). 14 days after the administration, the mice were intracerebrally (IC) infected with a suspension of virulent *B. pertussis* (strain 18323; SCLAVO S.p.A.) containing 300 average lethal doses. The mice were then kept under observation for 14 days. The results are reported in Table XIII.

**TABLE XIII**

| Cellular^{a} vaccine | | Acellular vaccine | | |
|---|---|---|---|---|
| | | PTF-9K/129G absorbed | PT-9K/129G fluid | |
| Dose ml | surv.^{b} | dose µg/mouse | surv.^{b} | surv.^{b} |
| 0.04 | 16/16 | 30 | 14/16 | 16/16 |
| 0.008 | 13/18 | 12 | N.D.^{c} | 16/16 |
| 0.0010 | 9/16 | 6 | 14/16 | N.D. |
| 0.00032 | 1/16 | 4.80 | N.D. | 12/16 |
| | | 1.92 | N.D. | 10/16 |
| | | 1.20 | 8/16 | 7/16 |
| | | 0.24 | 2/16 | 3/16 |
| | | PD₅₀ (d) | 1.2 | 1.1 |

| | | | | |
|---|---|---|---|---|
| "a" = vaccine contains eight protective international units/ml; | | | | |
| "b" = values are expressed as the number of surviving mice on a total of 16 mice tested; | | | | |
| "c" N.D. = non determined; | | | | |
| "d" dose which protected 50% of the mice from intracerebral infections with virulent *B. pertussis* 18323. | | | | |

As can be observed from the table, the PTF-9K/129G absorbed on A1(OH)₃ induced a protection from paralysis or death in 50% of mice at a dose of 1.2 µg/mouse (PD₅₀).

Better results were obtained with the fluid PT-9K/129G untreated with formaldehyde.

In fact, each time PT-9K/129G was utilized as immunogen, 100% protection was reached immediately after intracerebral challenge. The PD₅₀ (1.1 µg/mouse) did not, however, change in a significant way.

### Example 10

### Clinical Experimentation of the Acellular Antipertussis Vaccine in Adult Volunteers

The purpose of this study was to evaluate the tolerance and the immunogenicity (capacity of inducing specific neutralizing antibodies) of the acellular antipertussis vaccine containing PTF-9K/129G as an active component in a volunteer population.

For this purpose, 29 adult, healthy individuals of both sexes were selected, with anti-PT antibody titers lower than 20 ELISA units (EU)/ml. The patients were subdivided according to their anamnesis (unknown/negative for pertussis, positive for illness, positive for vaccination).

After random selection within each group, the patients are successively treated with:
1) antipertussis acellular vaccine containing the PTF-9K/129G mutant (18 volunteers).
   Each 0.5 ml dose contained 15 µg PTF-9K/129G (active component) 0.001 mg sodium-ethyl-mercury thiosalicylate and 0.5 mg aluminum hydroxide (excipient) or
2) placebo consisting of an aluminium hydroxide suspension of undifferentiated aspect vis a vis the vaccine (11 volunteers). On the basis of a random selection each patient was administered 2 doses antipertussis vaccine or 2 doses placebo, intramuscularly at the level of the left deltoid with an interval of 6 weeks between the 2 doses.

The patients were kept under observation for 3 months after the start of the experiment, and all the local and/or systemic side effects were noted.

The administration of whole plasma and/or human gamma globulins was avoided starting 3 months prior to the start of the experiment and for its entire duration.

In the same period of time, the administration of cortisone compounds and/or antihypertensive drugs was also avoided.

The patients were kept under strict observation for 30 minutes after the vaccine administration. Every day, for the 5 days successive to the vaccine administration, the values of the body temperature were taken by acellular measurement, and inspection and palpation of the administration site and of the satellite superficial lymphoglandular locations was performed.

4 days after the antipertussis vaccine administration, a sample of peripheral venous blood was taken from each patient for the purpose of carrying out the hematological, hematochemical and immunological tests (activation markers of lymphoid cells as CD3, CD4, CD19, CD25, CD23, CD14 and CD57; *in vitro* proliferation with respect to the PT, PT-9K/129G and tetanus toxoid antigens).

30 days after the vaccine administration, besides the antibody titration, tests for the cell mediated immunity evaluation and IgE titration were performed.

The same analyses were carried out according to the methods reported above after the second vaccine or placebo administration.

The absence of side effects after the first and second vaccine administration indicated a complete tolerance of the PTF-9K/129G mutant.

For each volunteer, the antipertussis antibody titer (ELISA assay) and the neutralizing one (CHO cells) were determined.

## Claims

1. An immunologically active pertussis toxin mutant having reduced or no toxicity characterised in that amino acid residue Glu129 and an amino acid residue or combination of residues selected from Arg9, Asp11, Arg13, Trp26, Phe50, Gly86, Ile88/Tyr89, Asp11/Trp26, Asp11/Phe50, Arg13/Trp26, Arg13/Phe50 and Tyr8/Arg9 are deleted or substituted by a different amino acid residue selected from the group of natural amino acids, excluding mutants having an Arg9 or Arg13 deletion or an Arg9 or Arg13 to Glu substitution in combination with a Glu129 or Glu129/Tyr130 substitution.

2. A pertussis toxin mutant according to claim 1, characterised in that the amino acid residue Glu129 and Arg9 are substituted respectively by amino acid residues Gly and Lys.

3. A pertussis toxin mutant according to claim 1, characterised in that amino acid residues Glu129 and Trp26 are substituted respectively by amino acid residues Gly and Ile.

4. A pertussis toxin mutant according to claim 1, characterised in that amino acid residues Glu129, Arg13 and Trp26 are substituted respectively by amino acid residues Gly, Leu and Ile.

5. A pertussis toxin mutant according to claim 1, characterised in that amino acid residues Glu129 and Arg13 are substituted respectively by amino acid residues Gly and Ile.

6. A pertussis toxin mutant according to claim 1, characterised in that amino acid residues Ile88, Tyr89 and Glu129 are substituted respectively by amino acid residues Glu, Ser and Gly.

7. A Bordetella strain characterised in that the Bordetella chromosome contains a gene coding for a pertussis toxin and expression and secretion regulation sequences wherein the S1 sub-unit nucleotide sequence codes for a pertussis toxin mutant according to any one of claims 1 to 6.

8. A Bordetella strain according to claim 7, in which Bordetella is *Bordetella pertussis*, *Bordetella parapertussis* or *Bordetella bronchiseptica*.

9. *Bordetella pertussis* (W28) PT-9K/129G (ATCC 53894).

10. *Bordetella parapertussis* PT-26I/129G (ATCC 53893).

11. A process for preparing a Bordetella strain according to any one of claims 7 to 10 comprising the steps:
a) selecting a wild type Bordetella strain resistant to at least one antibiotic;
b) substituting by homologous recombination in the strain obtained in (a) the chromosomal gene encoding for the pertussis toxin with a gene encoding a different protein;
c) selecting a Bordetella strain free of the PT (Δ tox) gene obtained in (b);
d) mutagenizing the pertussis toxin gene isolated from *B. pertussis* to produce a gene coding for a pertussis toxin mutant according to any one of claims 1 to 6;
e) inserting said mutagenized gene in a suitably modified plasmid non replicable in Bordetella;
f) inserting said plasmid in Bordetella strains (Δ tox) selected in (c) by conjunction and finally
g) isolating the Bordetella strains in which a homologous recombination with the mutagenized pertussis toxin gene has taken place.

12. A process according to claim 11, in which, in stage d) the pertussis toxin gene is isolated from plasmid PT-101 ATCC 67854.

13. A process for the preparation of a pertussis toxin mutant according to any one of claims 1 to 6, obtained by a process comprising: cultivating a Bordetella strain according to claims 7 to 10 and isolating and purifying the pertussis toxin mutant.

14. The use of a pertussis toxin mutant according to any one of claims 1 to 6, as the active component for the preparation of an acellular antipertussis vaccine capable of imparting to humans an effective protective immunity against infections caused by virulent *Bordetella pertussis*.

15. The use of Bordetella strains according to any one of claims 7 to 10 as the active component for the preparation of cellular antipertussis vaccines capable of imparting to humans an effective protective immunity against infections caused by virulent *Bordetella pertussis*.

16. An immunogenic formulation suitable as an acellular antipertussis vaccine capable of inducing in humans protective immunity against infections caused by virulent *Bordetella pertussis*, containing an immunologically effective amount of a pertussis toxin mutant according to each of claims 1 to 6.

17. An immunogenic formulation suitable as cellular antipertussis vaccine, capable of inducing in humans a protective immunity against infections caused by *Bordetella pertussis*, containing an immunologically effective amount of a Bordetella strain according to claims 7 to 10.

18. A process for the production of a pertussis toxin mutant as defined in any one of claims 1 to 6 comprising the step of treatment with formaldehyde at between 0.035% (w/v) and 0. 420% (w/v) to provide a thermally stable pertussis toxin mutant.

19. A gene coding for a pertussis toxin mutant as defined in any one of claims 1 to 6.

20. A plasmid containing the gene as defined in claim 19.

21. A microorganism transformed with a plasmid as defined in claim 20.

## Patentansprüche

1. Immunologisch wirksame Pertussistoxin-Mutante mit reduzierter oder keiner Toxizität, dadurch gekennzeichnet, daß der Aminosäurerest Glu129 und ein Aminosäurerest oder eine Kombination von Resten, ausgewählt aus Arg9, Asp11, Arg13, Trp26, Phe50, Gly86, Ile88/Tyr89, Asp11/Trp26, Asp11/Phe50, Arg13/Trp26, Arg13/Phe50 und Tyr8/Arg9, deletiert sind oder durch einen unterschiedlichen Aminosäurerest ersetzt sind, der ausgewählt ist aus den natürlichen Aminosäuren, ausschließlich Mutanten mit einer Arg9- oder Arg13-Deletion oder einem Ersatz von Arg9 oder Arg13 durch Glu in Kombination mit einem Ersatz von Glu129 oder Glu129/Tyr130.

2. Pertussistoxin-Mutante nach Anspruch 1, dadurch gekennzeichnet, daß die Aminosäurereste Glu129 und Arg9 durch die Aminosäurereste Gly bzw. Lys ersetzt sind.

3. Pertussistoxin-Mutante nach Anspruch 1, dadurch gekennzeichnet, daß die Aminosäurereste Glu129 und Trp26 durch die Aminosäurereste Gly bzw. Ile ersetzt sind.

4. Pertussistoxin-Mutante nach Anspruch 1, dadurch gekennzeichnet, daß die Aminosäurereste Glu129, Arg13 und Trp26 durch die Aminosäurereste Gly, Leu bzw. Ile ersetzt sind.

5. Pertussistoxin-Mutante nach Anspruch 1, dadurch gekennzeichnet, daß die Aminosäurereste Glu129 und Arg13 durch die Aminosäurereste Gly bzw. Ile ersetzt sind.

6. Pertussistoxin-Mutante nach Anspruch 1, dadurch gekennzeichnet, daß die Aminosäurereste Ile88, Tyr89 und Glu129 durch die Aminosäurereste Glu, Ser bzw. Gly ersetzt sind.

7. Bordetella-Stamm, dadurch gekennzeichnet, daß das Bordetella-Chromosom ein Gen enthält, das ein Pertussistoxin codiert, und Expressions- und Sekretions-Regulationssequenzen, wobei die S1-Untereinheit-Nucleotidsequenz eine Pertussistoxin-Mutante nach einem der Ansprüche 1 bis 6 codiert.

8. Bordetella-Stamm nach Anspruch 7, wobei Bordetella Bordetella pertussis, Bordetella parapertussis oder Bordetella bronchiseptica ist.

9. Bordetella pertussis (W28) PT-9K/129G (ATCC 53894).

10. Bordetella parapertussis PT-26I/129G (ATCC 53893).

11. Verfahren zur Herstellung eines Bordetella-Stamms nach einem der Ansprüche 7 bis 10, umfassend die folgenden Schritte:
a) Auswählen eines Wildtyp-Bordetella-Stamms, der resistent gegenüber mindestens einem Antibiotikum ist;
b) Ersetzen des chromosomalen Gens, das das Pertussisgen codiert, im in (a) erhaltenen Stamm mit einem Gen, das ein unterschiedliches Protein codiert, durch homologe Rekombination;
c) Auswählen eines Bordetella-Stamms, der frei ist von dem in (b) erhaltenen PT (Δ tox)-Gen;
d) Mutagenisierung des aus B. pertussis isolierten Pertussistoxin-Gens, wobei ein Gen produziert wird, das eine Pertussistoxin-Mutante nach einem der Ansprüche 1 bis 6 codiert;
e) Insertion des mutagenisierten Gens in ein geeignet modifiziertes Plasmid, das in Bordetella nicht replizierbar ist;
f) Insertion des Plasmids in Bordetella-Stämme (Δ tox), die in (c) ausgewählt wurden, durch Konjunktion und schließlich
g) Isolieren der Bordetella-Stämme, in denen eine homologe Rekombination mit dem mutagenisierten Pertussistoxin-Gen stattgefunden hat.

12. Verfahren nach Anspruch 11, wobei in Schritt d) das Pertussistoxin-Gen vom Plasmid PT-101 ATCC 67854 isoliert wurde.

13. Verfahren zur Herstellung einer Pertussistoxin-Mutante nach einem der Ansprüche 1 bis 6, erhalten durch folgendes Verfahren: Züchten eines Bordetella-Stammes nach den Ansprüchen 7 bis 10 und Isolieren und Reinigen der Pertussistoxin-Mutante.

14. Verwendung einer Pertussistoxin-Mutante nach einem der Ansprüche 1 bis 6 als Wirkstoff für die Herstellung eines zellulären anti-Pertussis-Impfstoffes, der Menschen eine wirksame Schutzimmunität gegen Infektionen durch virulente Bordetella pertussis verleihen kann.

15. Verwendung der Bordetella-Stämme nach einem der Ansprüche 7 bis 10 als Wirkstoff für die Herstellung von zellulären anti-Pertussis-Impfstoffen, die Menschen eine wirksame Schutzimmunität gegen Infektionen durch virulente Bordetella pertussis verleihen können.

16. Immunogene Formulierung, geeignet als azellulärer anti-Pertussis-Impfstoff, der in Menschen Schutzimmunität gegen Infektionen durch virulente Bordetella pertussis induzieren kann, enthaltend eine immunologisch wirksame Menge einer Pertussistoxin-Mutante nach jedem der Ansprüche 1 bis 6.

17. Immunogene Formulierung, geeignet als zellulärer anti-Pertussis-Impfstoff, der im Menschen eine Schutzimmunität gegen Infektionen durch Bordetella pertussis induzieren kann, enthaltend eine immunologisch wirksame Menge eines Bordetella Stamms nach den Ansprüchen 7 bis 10.

18. Verfahren zur Herstellung einer Pertussistoxin-Mutante nach der Definition in einem der Ansprüche 1 bis 6, umfassend den Schritt der Behandlung mit Formaldehyd zwischen 0,035 % (Gew./Vol.) und 0,420 % (Gew./Vol.) zum Erhalt einer thermisch stabilen Pertussistoxin-Mutante.

19. Gen, das eine Pertussistoxin-Mutante nach der Definition in einem der Ansprüche 1 bis 6 codiert.

20. Plasmid, enthaltend das Gen gemäß der Definition in Anspruch 19.

21. Mikroorganismus, transformiert mit einem Plasmid gemäß der Definition in Anspruch 20.

## Revendications

1. Toxine mutante de pertussis immunologiquement active n'ayant pas ou peu de toxicité, caractérisée en ce que le résidu amino acide Glu129 et un résidu amino acide ou une combinaison de résidus sélectionnés parmi Arg9, Asp11, Arg13, Trp26, Phe50, Gly86, Ile88/Tyr89, Asp11/Trp26, Asp11/Phe50, Arg13/Tpr26, Arg13/Phe50 et Tyr8/Arg9 sont délétés ou substitués par un résidu amino acide différent sélectionné parmi le groupe des amino acides naturels à l'exclusion des mutants ayant une délétion Arg9 ou Arg13 ou une substitution Arg9 ou Arg13 du Glu en combinaison avec une substitution Glu 129 ou Glu129/Tyr130.

2. Toxine mutante de pertussis selon la revendication 1 caractérisée en ce que les résidus amino acides Glu 129 et Arg9 sont substitués respectivement par les résidus amino acides Gly et Lys.

3. Toxine mutante de pertussis selon la revendication 1, caractérisée en ce que les résidus amino acides Glu129 et Trp26 sont substitués respectivement par des résidus amino acides Gly et Ile.

4. Toxine mutante de pertussis selon la revendication 1, caractérisée en ce que les résidus amino acides Glu129, Arg13 et Trp26 sont substitués respectivement par des résidus amino acides Gly, Leu et Ile.

5. Toxine mutante de pertussis selon la revendication 1, caractérisée en ce que les résidus amino acides Glu129 et Arg13 sont substitués respectivement par des résidus amino acides Gly et Ile.

6. Toxine mutante de pertussis selon la revendication 1, caractérisée en ce que les résidus amino acides Ile88, Tyr89 et Glu129 sont substitués respectivement par des résidus amino acides Glu, Ser et Gly.

7. Souche de Bordettella caractérisée en ce que le chromosome de Bordetella contient un gène codant pour une toxine de pertussis et des séquences régulatrices d'expression et de sécrétion, dans laquelle la séquence nucléotide sous unité S1 code pour une toxine mutante de pertussis selon l'une quelconque des revendications 1 à 6.

8. Souche de Bordetella selon la revendication 7, dans laquelle Bordetella est *Bordetella pertussis*, *Bordetella parapertussis* ou *Bordetella bronchiseptica*.

9. *Bordetella pertussis* (W28) PT-9K/129G (ATCC 53894).

10. *Bordetella parapertussis* PT-26I/129G (ATCC 53893).

11. Procédé de préparation d'une souche de Bordetella selon l'une des revendications 7 à 10, comprenant les étapes de :
a) sélection d'une souche de Bordetella de type sauvage résistant à au moins un antibiotique;
b) substitution par recombinaison homologue dans la souche obtenue en a) du gène chromosomique codant pour la toxine de pertussis avec un gène codant une protéine différente;
c) sélection d'une souche de Bordetella libre du gène PT (Δ tox) obtenu en b);
d) mutagénèse du gène de toxine de pertussis à partir de *B. pertussis* pour obtenir un gène codant pour une toxine de pertussis mutante selon l'une quelconque des revendications 1 à 6;
e) insertion dudit gène mutagéné dans un plasmide modifié approprié non reproductible dans Bordetella;
f) insertion dudit plasmide dans les souches de Bordetella (Δ tox) sélectionnées en c) par conjonction, et finalement
g) isolation des souches de Bordetella dans lesquelles une recombinaison homologue avec le gène mutagéné de la toxine de pertussis a pris place.

12. Procédé selon la revendication 11, dans lequel, à l'étape d) le gène de la toxine de pertussis est isolé du plasmide PT-101 ATCC 67854.

13. Procédé pour la préparation d'une toxine mutante de pertussis selon l'une quelconque des revendications 1 à 6, obtenue par un procédé comprenant : la culture d'une souche de Bordetella selon l'une des revendication 7 à 10 et l'isolation et la purification de la toxine mutante de pertussis.

14. Utilisation d'une toxine mutante de pertussis selon l'une quelconque des revendications 1 à 6, comme composant actif pour la préparation de vaccin non cellulaire antipertussis capable de produire chez les humains une immunité protectrice efficace contre les infections causées par *Bordetella pertussis* virulent.

15. Utilisation de souches de Bordetella selon l'une quelconque des revendications 7 à 10, comme composant actif pour la préparation de vaccins cellulaires antipertussis capables d'induire chez les humains une immunité protectrice contre les infections causées par *Bordetella pertussis* virulent.

16. Formulation immunogène utile en tant que vaccin antipertussis non cellulaire, capable d'induire chez l'homme une immunité protectrice contre les infections causées par *Bordetella pertussis* virulent, contenant une quantité immunologique efficace d'une toxine mutante de pertussis selon l'une des revendications 1 à 6.

17. Formulation immunogène utile comme vaccin antipertussis cellulaire, capable d'induire chez l'homme une immunité protectrice contre les infections causées par *Bordetella pertussis*, contenant une quantité immunologique efficace d'une souche de Bordetella selon l'une quelconque des revendications 7 à 10.

18. Procédé pour la préparation d'une toxine mutante de pertussis telle que définie dans l'une quelconque des revendications 1 à 6, comprenant l'étape de traitement avec du formaldehyde à entre 0,035% (P/V) et 0,420% (P/V) pour obtenir une toxine mutante de pertussis thermiquement stable.

19. Gène codant pour une toxine mutant de pertussis telle que définie selon l'une quelconque des revendications 1 à 6.

20. Plasmide contenant le gène tel que défini par la revendication 19.

21. Microorganisme transformé avec un plasmide tel que défini dans la revendication 20.
